(19) Europäisches Patentamt
European Patent Office
Office européen des brevets

(11) **EP 4 099 987 B1**

(12) **EUROPEAN PATENT SPECIFICATION**

(45) Date of publication and mention of the grant of the patent:
**12.03.2025 Bulletin 2025/11**

(21) Application number: **21703212.7**

(22) Date of filing: **02.02.2021**

(51) International Patent Classification (IPC):
***A61K 9/107*** *(2006.01)* ***A61K 9/14*** *(2006.01)*
***A61K 47/20*** *(2006.01)* ***A61K 47/34*** *(2017.01)*
***A61K 47/38*** *(2006.01)* ***A61K 31/506*** *(2006.01)*
***A61P 9/00*** *(2006.01)*

(52) Cooperative Patent Classification (CPC):
**A61K 9/1075; A61K 9/146; A61K 31/506; A61K 47/20; A61K 47/34; A61K 47/38; A61P 9/00**

(86) International application number:
**PCT/EP2021/052362**

(87) International publication number:
**WO 2021/156223 (12.08.2021 Gazette 2021/32)**

(54) **NANOFORMULATIONS OF METHYL {4,6-DIAMINO-2-[5-FLUORO-1-(2-FLUOROBENZYL)-1H-PYRAZOLO[3,4-B]PYRIDIN-3-YL] PYRIMIDIN-5-YL}CARBAMATE**

NANOFORMULIERUNGEN VON METHYL {4,6-DIAMINO-2-[5-FLUOR-1-(2-FLUORBENZYL)-1H-PYRAZOLO[3,4-B]PYRIDIN-3-YL] PYRIMIDIN-5-YL}CARBAMAT

NANOFORMULATIONS DE MÉTHYL {4,6-DIAMINO-2-[5-FLUORO-1-(2-FLUOROBENZYL)-1H-PYRAZOLO[3,4- B]PYRIDIN-3-YL] PYRIMIDIN-5-YL}CARBAMATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.02.2020 EP 20155184**

(43) Date of publication of application:
**14.12.2022 Bulletin 2022/50**

(73) Proprietor: **Adverio Pharma GmbH**
**51373 Leverkusen (DE)**

(72) Inventors:
- **KERSTEN, Elisabeth**
  **42105 Wuppertal (DE)**
- **OSTENDORF, Michael**
  **51375 Leverkusen (DE)**
- **HOHEISEL, Werner**
  **51061 Köln (DE)**
- **NEUMANN, Heike**
  **42117 Wuppertal (DE)**
- **SOWA, Michal**
  **42109 Wuppertal (DE)**
- **BROCKOB, Joerg**
  **51379 Leverkusen (DE)**
- **FEY, Peter**
  **42111 Wuppertal (DE)**
- **LONGERICH, Markus**
  **50825 Köln (DE)**
- **BECKER, Guido**
  **47800 Krefeld (DE)**
- **CONTY, Valentina, Paula**
  **12103 Berlin (DE)**
- **EHRIG, Anja**
  **51373 Leverkusen (DE)**

(74) Representative: **BIP Patents**
**c/o Bayer Intellectual Property GmbH**
**Alfred-Nobel-Straße 50**
**40789 Monheim am Rhein (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
**WO-A1-2011/147809** **WO-A1-2013/076168**
**WO-A1-2020/014504** **WO-A1-2020/126983**
**CN-A- 108 721 296**

- **MARKUS FOLLMANN ET AL: "Discovery of the Soluble Guanylate Cyclase Stimulator Vericiguat (BAY 1021189) for the Treatment of Chronic Heart Failure", JOURNAL OF MEDICINAL CHEMISTRY, vol. 60, no. 12, 12 June 2017 (2017-06-12), US, pages 5146 - 5161, XP055441666, ISSN: 0022-2623, DOI: 10.1021/acs.jmedchem.7b00449**

## Description

The present invention relates to stable nanosuspensions of methyl {4,6-diamino-2-[5-fluoro-1-(2-fluorobenzyl)-1H-pyrazolo[3,4-b]pyridin-3-yl]pyrimidin-5-yl}carbamate (Vericiguat, compound of formula (I)), processes for preparing the stable nanosuspensions, nanoparticles comprising the compound of formula (I), and pharmaceutical compositions in solid form made from the nanosuspensions.

The number of poorly water-soluble drug candidates coming out of drug discovery has increased tremendously over the past few decades. Their formulation into efficacious dosage forms presents various challenges.

Methyl {4,6-diamino-2-[5-fluoro-1-(2-fluorobenzyl)-1H-pyrazolo[3,4-b]pyridin-3-yl]pyrimidin-5-yl}carbamate of formula (I)

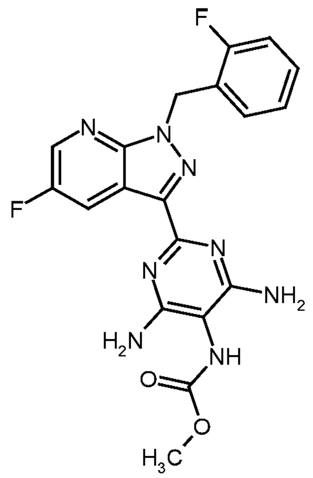

(I), (Vericiguat),

known from WO 2011/147809 is one of these active pharmaceutical ingredients (API) with limited dissolution behaviour affecting bioavailability. WO 2011/147809 A1 mentions solid and liquid formulations, including suspensions for oral administration. WO 2011/147809 A1 does not address the topic of limited dissolution behaviour of the compound of formula (I) nor any measures for increasing its dissolution. Further, WO 2011/147809 does not disclose crystalline forms of the compound of formula (I).

WO 2013/076168 A1 pertains inter alia to a process for manufacturing the compound of formula (I) and to intermediates used in this process. WO 2013/076168 A1 further pertains to the compound of formula (I) in the crystalline form of modification I and to crystalline substance compound of the formula (I) in the form of the di-dimethyl sulfoxide solvate of the compound of formula (I). The di-dimethyl sulfoxide solvate of the compound of formula (I) is used as intermediate in the process for obtaining the compound of formula (I) in the crystalline form of modification I in high purity. WO 2013/076168 A1 further mentions the stability of the compound of formula (I) in the crystalline form of modification I which is preserved during micronization, meaning that no conversion and recrystallization takes place. WO 2013/076168 A1 does not address the topic of limited dissolution behaviour of the compound of formula (I) nor any measures for increasing its dissolution.

The publication Follmann et al. (2017) pertains to the discovery of the compound of the formula (I) for the treatment of heart failure. Follmann et al. describe the process steps for manufacturing the compound of the formula (I) in the crystalline form of modification I, including the final step as described in Example 13 of WO 2013/076168 A1 using the di-dimethyl sulfoxide solvate of the compound of formula (I) as intermediate. This process yields the highly purified compound of formula (I) in the crystalline form of modification I in dry form.

WO 2020/126983 A1, published after the first filing date of the present application, pertains for example to an active compound product of the compound of formula (I) in the crystalline form of modification I having improved properties, for example with respect to the isolability of the active compound product, the dischargeability of the active compound product after isolation and drying and also conveyability, sieveability and micronizability of the active compound product. These improved properties allow to run the manufacturing process on a technical scale. Improved micronization is said to be measurable for example via easier feeding of the active compound product into the jet mill. It is further mentioned that in the context of WO 2020/126983 A1, micronization is carried out for example by comminution in a jet mill. Jet mills perform dry milling. Jet mills are suitable for grinding of particles down to the micrometer range. The grinding

action in a jet mill is created by the high-velocity collisions between particles driven by multiple jets of air or steam. Dry milling is not suitable for producing nanoparticles. WO 2020/126983 A1 does not address the topic of limited dissolution behaviour of the compound of formula (I) nor any measures for increasing its dissolution.

WO 2020/014504 A1 pertains to the use of sGC stimulators, including vericiguat, for the treatment of mitochondrial disorders. WO 2020/014504 A1 further pertains to formulations of sGC stimulators in general, mentioning inter alia stabilizers and nanoparticles. WO 2020/014504 A1 however does not provide any specific teaching of stable nanoformulations of vericiguat or how to prepare them, except the general statement that the formulations may be prepared using conventional dissolution and mixing procedures.

CN 108721296 A pertains to the use of Vericiguat for treating high altitude disease. In example 1, CN 108721296 A describes certain compositions of solid Vericiguat but does not disclose crystalline forms or nanoformulations of Vericiguat.

The envisaged clinical dose for methyl {4,6-diamino-2-[5-fluoro-1-(2-fluorobenzyl)-1H-pyrazolo[3,4-b]pyridin-3-yl]pyrimidin-5-yl}carbamate (Vericiguat, compound of formula (I)) ranges from 2.5-15 mg once daily, depending on the targeted indication. A standard immediate release tablet was developed. With increasing doses, a decrease in bioavailability with standard immediate release formulations like tablets and a positive food effect, shown as an increase of bioavailability with food intake, is observed.

Food can impact the pharmacokinetics of a drug product through several mechanisms, such as delay in gastric emptying, stimulation of bile flow, changes in gastrointestinal (GI) pH, alterations in luminal metabolism, or interactions of the drug with the food itself. The drug absorption process can be affected by many factors, including calorie content (low vs high calorie meals), nutrient composition (protein, carbohydrate-rich or high-fat meals), volume, temperature of the meal itself, and fluid ingestion. Food also increases blood flow to the liver (splanchnic blood flow); therefore, changes in first pass extraction that occur as a result, may cause differences in bioavailability between the fed and fasted state.

Preparation of drug nanoparticles or nanocrystals is one way to formulate such drugs with limited dissolution behaviour because size reduction of drug crystals increases the specific surface area, which can improve the dissolution velocity of such drugs and, in turn, their bioavailability. Moreover, ultrafine particles tend to show higher saturation solubility, which also enhances the dissolution velocity.

Within the meaning of the present invention, a sufficient dissolution velocity is defined as the capacity that enough drug particles of the envisioned dose range could be dissolved within the transit time at the absorption site. In contrast, a drug has a limited dissolution velocity if the velocity of dissolution is too slow for all of the drug particles to dissolve during the time for transit past the absorption site (Butler, Dressman, 2010).

There are several approaches to producing drug nanoparticles such as wet bead milling (also referred to as nanomilling in the context of nanosuspension preparation), homogenization, liquid antisolvent precipitation, melt emulsification, precipitation using supercritical fluid, evaporative precipitation, and micro-emulsions.

Particle size during milling generally depends on (i) process-equipment parameters, including specific energy input and stress intensity; (ii) mechanical and physico-chemical properties of drug particles; and (iii) physical stability of the milled suspension, i.e., mitigation of aggregation and/or Ostwald ripening in the presence of various stabilizers. "Stress intensity (SI)" is defined according to Kwade et al. (1996), as

$$SI \propto SI_b = d_b^3 \cdot (\rho_b - \rho) \cdot v_d^2$$

with SI = stress intensity; $SI_b$ = stress intensity of the beads; $d_b$ = diameter of the grinding beads [m]; $\rho_b$ = densitiy of the grinding beads; $\rho$ = density of the fluid [kg/m$^3$]; $v_d$ = circumferential speed of the stirrer discs [m/s].

"Ostwald ripening" describes the phenomenon in which smaller particles in solution dissolve and deposit on larger particles in order to reach a more thermodynamically stable state by minimizing the surface to area ratio, fostering the formation of larger particles. Preparation of a drug nanosuspension with desired particle size and adequate storage stability entails a wet bead milling (or nanomilling) process, as defined below and described in Examples 1 to 3. The selection of optimal stabilizer formulation is a laborious and resource-demanding task, yet an important one with potentially serious consequences. A poorly formulated nanosuspension of a drug may undergo aggregation, Ostwald ripening, fast sedimentation of particles, and cake formation during milling/storage, which will lead to various issues in downstream processing of the respective suspensions and poor product performance of the final dosages such as unexpectedly slow dissolution velocity.

According to Kwade et al (1996), an optimum stress intensity exists for a fixed specific energy input, for which the finest product is achieved. With increasing specific energy input, and therefore increasing product fineness, the optimum stress intensity decreases. Since the specific energy is proportional to the product of stress intensity and stress frequency, the comminution result can also be correlated to the stress frequency and the stress intensity. With increasing stress intensity, the stress frequency required for a certain product fineness decreases.

Potential particle size increase or particle growth during milling and storage can lead to loss of high surface area

associated with the drug nanoparticles, which reduces the significant benefits intended from the nanomilling process.

Stabilization of nanoparticles in solution is always necessary in order to utilize their specific properties. Agglomeration, growth or fusion have to be avoided over time. Due to their high specific surface area nanoparticles are always in a "high energy state" and therefore not intrinsically stable. Therefore, special measures must be taken for stabilizing nanoparticle suspensions.

Stabilization of drug nanosuspensions produced via wet bead milling can be achieved by various excipients depending on the underlying stabilizing mechanism, which cannot be anticipated. The data in prior art suggest that wet bead milling has been used effectively to prepare nanosuspensions of a multitude of poorly water-soluble drugs, and various polymers and/or surfactants can be used for ensuring the adequate physical stability of the nanosuspensions. Interestingly, only a few nanosuspensions achieved final drug particle sizes below 100 nm. Hence, there is a huge gap in pharmaceutical nanotechnology literature regarding the preparation of drug nanoparticles via wet bead milling.

As outlined in a review by Li et al. (2016), preparation of drug nanoparticles via wet bead milling (nanomilling) is a well-known and established technology for oral and other forms of applications. According to Li et al. (2016), wet bead milling followed by various drying processes has become a wellestablished and proven formulation approach especially for bioavailability enhancement of poorly water-soluble drugs. The physical stability of wet-milled suspensions (nanosuspensions) is said to have attracted a lot of attention, while the fundamental understanding of the process is still lacking. The selection of surfactants and their optimum concentration is critical regarding physical instability and is a laborious and resource-demanding task. A first principle-based predictive method to select proper stabilizer(s) for a given drug is still missing. It is concluded that no correlation between physicochemical drug properties (molecular weight, melting point, logP, solubility and density) and stable nanosuspension formation exists. Inadequate concentration of stabilizers such as polymers or surfactants may not prevent drug nanoparticle aggregation, while its excess (especially if the concentration of surfactant is above the critical micelle concentration (CMC)) is considered to accelerate Ostwald ripening. Solid state changes upon milling may influence bioavailability and further manufacturing. Also, the bridging from one mill to another might be critical.

Kumar et al. (2008) point out the influence and importance of identifying right stabilizer (s) and process parameters such as influence of number of homogenizing cycles on particle size, and sequence of mixing of ingredients on the physical characteristics of nanosuspensions. George & Gosh (2013) analyse the mechanism of stabilization as a function of drug properties and conclude that the optimization of nanosuspension using media milling approach is a complex process since it involves many factors that affects the characteristics of the nanosuspension product.

According to Desai (2012), the major challenges in designing nanosuspensions for oral delivery are maintaining colloidal stability and particle size of the nanosuspensions during storage and in the gastrointestinal tract after oral administration and conversion of the nanosuspensions to a palatable and patient-friendly oral formulation.

Publications by Choi & Han (2018) and Jermain et al. (2018) provide recent updates on nanocrystal technologies for poorly water-soluble drugs. As summarized by Jermain et al. (2018), nanoparticles are much more unstable than microparticles because of the extra Gibbs free energy contribution related to reducing particle size and primarily due to increased surface energy. Addressing this extra contribution is key to formulating pharmaceutical nanoparticles because they will tend to agglomerate to minimize their total energy (Van Eerdenbrugh et al., 2008). As outlined by Jermain et al. (2018), thermodynamic stabilization may be used as approach to stabilize drug nanoparticles which uses surfactants or block copolymers for particle stability. For maximum effectiveness, the two approaches are often combined (Lee et al., 2008). Careful selection of the amount of stabilizer is as important as the selection of the type of stabilizer. For example, one obstacle to stabilization is Ostwald ripening, which is described above. Too little stabilizer allows agglomeration of nanoparticles and too much stabilizer promotes Ostwald ripening (Merisko-Liversidge et al., 2003).

Several marketed products are known which are based on nanoformulations, manufactured via wet bead milling (table modified after book "Nanomedicine in Health and Disease" (Hunter, 2011):

| Trade name | API | Company | Reason for using nanosized API | Patent |
|---|---|---|---|---|
| Rapamune® | Sirolimus | Wyeth | Reformulation, patient-friendly tablet instead of solution | US Patent No. 5,145,684; |
| Emend® | Aprepitant | Merck | NCE, high bioavailability, no food effects | US Patent No. 8,258,132, |
| TriCor® Lyphantyl® | Fenofibrate | Fournier Pharma, Abbott Laboratories | Reformulation, no food effects | US Patent Nos. 6,375,986; 7,276,249; and 7,320,802 |

(continued)

| Trade name | API | Company | Reason for using nanosized API | Patent |
|---|---|---|---|---|
| Megace ES® | Megestrol acetate | PAR pharmaceuticals | Reformulation, no food effects, more patient-friendly | US Patent Nos. 6,592,903 and 9,101,540 |

As outlined above, common technical problems with regard to nanoformulations that remain to be solved for each individual compound include:

a) Choice of stabilizer

b) Mechanical Processing: impact of process parameters (such as choice of mill, specific energy input and stress intensity, duration of milling, tip speed), material of milling beads, size of milling beads, volumetric filling of milling beads, and physicochemical properties of the drug.

c) Further processing: risk of loss of beneficial effects based on nano-size upon drying of the nanosuspension e.g. due to agglomeration or Ostwald ripening or further processing to final dosage form storage, and resuspension at the site of absorption.

In view of the prior art, one object of the present invention is to solve the problem that due to the limited dissolution behaviour of Vericiguat, bioavailability in preclinical and clinical studies may decrease with higher doses.

This object of the present invention is solved by providing the stable nanosuspensions of the compound of formula (I) according to the invention, processes for preparing the stable nanosuspensions, nanoparticles comprising the compound of formula (I), and pharmaceutical compositions in solid form made from the nanosuspensions, by which the bioavailability that is limited by a low dissolution velocity could be increased in general and a food effect is expected to be circumvented.

According to an embodiment, the present invention provides stable nanosuspensions of the compound of formula (I) (vericiguat). For vericiguat, no stable nanoformulations have been described before.

One embodiment of the present invention is a stable nanosuspension comprising nanoparticles of the compound of formula (I) in crystalline form of modification I and one or more stabilizer(s) in a dispersing agent, wherein the maximum concentration of the one or more stabilizer(s) is the solubility limit of the stabilizer(s) in the suspension and wherein said nanoparticles have an average particle size, expressed as d50, of 500 nm or less.

Within the meaning of the present invention, the compound of formula (I) is methyl {4,6-diamino-2-[5-fluoro-1-(2-fluorobenzyl)-1H-pyrazolo[3,4-b]pyridin-3-yl]pyrimidin-5-yl}carbamate

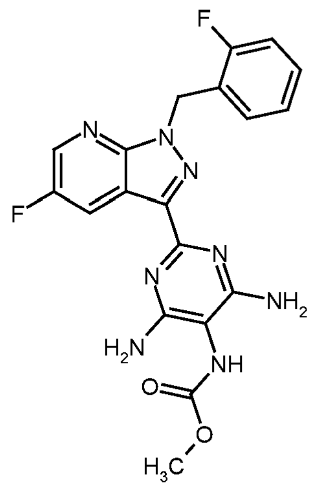

(I).

Within the meaning of the present invention, "compound of formula (I) in crystalline form of modification (I)" is to be understood as meaning the modification of the compound of formula (I) which is defined as crystalline modification I in WO 2013/076168; for example by reference to the x-ray diffractogram having defined peak maxima of the 2 theta angle at 5.9, 6.9 and 22.7 or at 5.9, 6.9, 16.2, 16.5, 24.1, 22.7 and 24.7; or via the IR spectrum having defined band maxima at 1707, 1633 and 1475 $cm^{-1}$ or at 1707, 1633, 1566, 1475, 1255 and 1223 $cm^{-1}$; or with the aid of the melting point of 257°C.

Within the meaning of the present invention, "drug nanoparticles" or "nanoparticles of the compound of formula (I) in crystalline form of modification I" are defined as particles of an active pharmaceutical ingredient (API) or of the compound of formula (I) in crystalline form of modification I, respectively, having a particle size, expressed as d50, at 500 nm or less, or at 400 nm or less, or at 300 nm or less, or at 250 nm or less, or at 200 nm or less, or at 150 nm or less, or at 100 nm or less.

Within the meaning of the present invention, particle size distribution is expressed as d-values. d-values can be thought of as the diameter of the sphere which divides the sample's mass into a specified percentage when the particles are arranged on an ascending mass basis. For example, the d10 is the diameter at which 10% of the sample's mass is comprised of particles with a diameter less than this value. The d50 is the diameter of the particle that 50% of a sample's mass is smaller than and 50% of a sample's mass is larger than. The d90 is the diameter of the particle that 90% of a sample's mass is smaller than and 10% of a sample's mass is larger than. The size of drug nanoparticles can be measured e.g. by dynamic light scattering (DLS), performed with a Zetasizer Nano-ZS (from Malvern Panalytical) or static light scattering (SLS), performed with a Mastersizer 3000 (from Malvern Panalytical).

Within the meaning of the present invention, a "stable nanosuspension" is defined as a nanosuspension comprising nanoparticles of the compound of formula (I) in crystalline form of modification I and one or more stabilizer(s) in a dispersing agent, wherein said nanoparticles have an average particle size, expressed as d50, of 500 nm or less, or 300 nm or less, or 250 nm or less or 200 nm or less, or 150 nm or less or 100 nm or less, wherein the average particle size, expressed as d50, remains at 500 nm or less , or 300 nm or less, or 250 nm or less or 200 nm or less, or 150 nm or less or 100 nm or less at storage for at least one week at a temperature of at least 40°C.

Within the meaning of the present invention, a "stable nanosuspension" is further defined as a nanosuspension comprising nanoparticles of the compound of formula (I) in crystalline form of modification I and one or more stabilizer in a ratio of 8:1 to 1:1 w/w, said nanoparticles having an average particle size, expressed as d50, of 500 nm or less, wherein said particle size of the nanoparticles, measured either with DLS or with SLS remains at 500 nm or less at storage for one or more weeks at 40°C. The long-term measurement of particle growth is shown in Example 2b and Fig. 2.

Within the meaning of the present invention, "stabilizers" are defined as substances and combinations thereof which improve the milling properties, cause the wetting of the surface and stabilization at the surface, and lead to physical stability of nanosuspensions.

Subject of the disclosure are stable nanosuspensions, wherein the one or more stabilizer is selected from the group consisting of polyvinylpyrrolidone (PVP), vinylpyrrolidone-vinyl acetate copolymer, ethylene oxide-propylene oxide block copolymer, sodium dodecylsulfate (SDS), hydroxypropylmethylcellulose (HPMC), d-alpha tocopheryl polyethylene glycol 1000 succinate (Vitamin E-TPGS), polysorbat, hydroxypropylcellulose (HPC), polyoxyl-35 castor oil, polyoxyl 15 hydroxystearate, Na-desoxycholate, and combinations thereof.

Subject of the disclosure are stable nanosuspensions, wherein the one or more stabilizer is selected from the group consisting of polyvinylpyrrolidone (PVP), vinylpyrrolidone-vinyl acetate copolymer (e.g. PVP VA 64), ethylene oxide-propylene oxide block copolymer (e.g. Poloxamer 188), sodium dodecylsulfate (SDS), hydroxypropylmethylcellulose (HPMC), d-alpha tocopheryl polyethylene glycol 1000 succinate (Vitamin E-TPGS), polysorbat (Tween®), hydroxypropylcellulose (HPC), polyoxyl-35 castor oil (Cremophor EL®), polyoxyl 15 hydroxystearate (Solutol HS 15®), Na-desoxycholate, and combinations thereof.

Subject of the disclosure are stable nanosuspensions, wherein the one or more stabilizer is selected from the group consisting of sodium dodecylsulfate, polyvinylpyrrolidone K10 to K50, hydroxypropylmethylcellulose, Vitamin E TPGS, polysorbat 20-80, and combinations thereof.

One embodiment of the present invention is a stable nanosuspension according to the invention, wherein the one or more stabilizer is selected from the group consisting of polyvinylpyrrolidone (PVP) in combination with sodium dodecylsulfate (SDS), vinylpyrrolidone-vinyl acetate copolymer, ethylene oxide-propylene oxide block copolymer, sodium dodecylsulfate (SDS), hydroxypropylmethylcellulose (HPMC), polysorbate, hydroxypropylcellulose (HPC), polyoxyl-35 castor oil, polyoxyl 15 hydroxystearate, Na-desoxycholate, and combinations thereof.

One embodiment of the present invention is a stable nanosuspension according to the invention, wherein the one or more stabilizer is selected from the group consisting of sodium dodecylsulfate, polyvinylpyrrolidone K10 to K50 in combination with sodium dodecylsulfate (SDS), hydroxypropylmethylcellulose, polysorbate 20-80, and combinations thereof.

According to an embodiment of the present invention, polyvinylpyrrolidone (PVP) is selected from PVP K10 to K50. According to a further embodiment of the present invention, polyvinylpyrrolidone (PVP) is selected from PVP K12 to K30. According to a further embodiment of the present invention, polyvinylpyrrolidone (PVP) is selected from PVP K12, PVP K17, and PVP K30. According to an embodiment of the present invention, vinylpyrrolidone-vinyl acetate copolymer is PVP VA 64. According to an embodiment of the present invention, ethylene oxide-propylene oxide block copolymer is Poloxamer 188. According to an embodiment of the present invention, polysorbat is selected from polysorbat 20-80. According to an embodiment of the present invention, polysorbat is polysorbat 80.

According to an embodiment of the present invention, the one or more stabilizer comprised in the nanoparticles

according to the present invention is a combination of SDS and PVP K12. According to an embodiment of the present invention, the one or more stabilizer comprised in the nanoparticles according to the present invention is a combination of SDS and PVP K17. According to an embodiment of the present invention, the one or more stabilizer comprised in the nanoparticles according to the present invention is a combination of SDS and Poloxamer 188.

One embodiment of the present invention are stable nanosuspensions according to the invention, wherein the one or more stabilizer is sodium dodecylsulfate. One embodiment of the present invention are stable nanosuspensions according to the invention, wherein the one or more stabilizer is polysorbat 20-80. One embodiment of the present invention are stable nanosuspensions according to the invention, wherein the one or more stabilizer is polysorbat 80.

Within the meaning of the present invention, the maximum concentration of the one or more stabilizer(s) is the solubility limit of the stabilizer(s) in the suspension. For pharmaceutical compositions made with the nanosuspensions of the present invention, the maximum concentration of the one or more stabilizer(s) is further limited by the acceptable daily intake (ADI), in case such ADI is defined for the respective stabilizer. According to the European Food Safety authority, the ADI is an estimate of the amount of a substance in food or drinking water that can be consumed daily over a lifetime without presenting an appreciable risk to health. It is usually expressed as milligrams of the substance per kilogram of body weight and applies to chemical substances such as food additives, pesticide residues and veterinary drugs.

Within the meaning of the present invention, "dispersing agent" is defined as a polar liquid in which the compound of formula (I) is insoluble. Examples include but are not limited to water; alcohols such as primary, secondary and tertiary alcohols including ethanol, propanol, isopropanol, butanol, isobutanol, tert. butanol; and polyvalent alcohols including glycerol. According to one embodiment of the invention, water is used as dispersing agent.

One embodiment of the present invention is a stable nanosuspension comprising nanoparticles of the compound of formula (I) in crystalline form of modification I and one or more stabilizer(s) in a dispersing agent, wherein the maximum concentration of the one or more stabilizer(s) is the solubility limit of the stabilizer(s) in the suspension and wherein said nanoparticles have an average particle size, expressed as d50, of 400 nm or less.

One embodiment of the present invention is a stable nanosuspension comprising nanoparticles of the compound of formula (I) in crystalline form of modification I and one or more stabilizer(s) in a dispersing agent, wherein the maximum concentration of the one or more stabilizer(s) is the solubility limit of the stabilizer(s) in the suspension and wherein said nanoparticles have an average particle size, expressed as d50, of 300 nm or less.

One embodiment of the present invention is a stable nanosuspension comprising nanoparticles of the compound of formula (I) in crystalline form of modification I and one or more stabilizer(s) in a dispersing agent, wherein the maximum concentration of the one or more stabilizer(s) is the solubility limit of the stabilizer(s) in the suspension and wherein said nanoparticles have an average particle size, expressed as d50, of 250 nm or less.

One embodiment of the present invention is a stable nanosuspension comprising nanoparticles of the compound of formula (I) in crystalline form of modification I and one or more stabilizer(s) in a dispersing agent, wherein the maximum concentration of the one or more stabilizer(s) is the solubility limit of the stabilizer(s) in the suspension and wherein said nanoparticles have an average particle size, expressed as d50, of 200 nm or less.

One embodiment of the present invention is a stable nanosuspension comprising nanoparticles of the compound of formula (I) in crystalline form of modification I and one or more stabilizer(s) in a dispersing agent, wherein the maximum concentration of the one or more stabilizer(s) is the solubility limit of the stabilizer(s) in the suspension and wherein said nanoparticles have an average particle size, expressed as d50, of 150 nm or less.

One embodiment of the present invention is a stable nanosuspension comprising nanoparticles of the compound of formula (I) in crystalline form of modification I and one or more stabilizer(s) in a dispersing agent, wherein the maximum concentration of the one or more stabilizer(s) is the solubility limit of the stabilizer(s) in the suspension and wherein said nanoparticles have an average particle size, expressed as d50, of 100 nm or less.

One embodiment of the present invention is a stable nanosuspension comprising nanoparticles of the compound of formula (I) in crystalline form of modification I and one or more stabilizer(s) in a dispersing agent, wherein the maximum concentration of the one or more stabilizer(s) is the solubility limit of the stabilizer(s) in the suspension and wherein said nanoparticles have an average particle size, expressed as d50, of 500 nm or less, and wherein the ratio of the compound of formula (I):one or more stabilizer(s) is 16:1 to 1:2 w/w.

In the case that a combination of two stabilizers is used and these two stabilizers are a surfactant and a polymer (e.g. SDS plus polyvinylpyrrolidone or SDS plus ethylene oxide-propylene oxide block copolymer), the ratio of compound (I):polymer is 16:1 to 1:2 w/w or 8:1 to 2:1 w/w and the concentration of the surfactant is 0.1-0.2% w/v.

One embodiment of the present invention are stable nanosuspensions of the invention, wherein the average particle size, expressed as d50, remains at 300 nm or less at storage for at least one week at a temperature of at least 40°C.

One embodiment of the present invention is a stable nanosuspension according to the present invention, wherein the one or more stabilizer is used at a ratio of the compound of formula (I): stabilizer of 8:1 to 2:1 w/w.

According to an embodiment of the present invention, the average particle size of the stable nanosuspension comprising nanoparticles of the compound of formula (I) in crystalline form of modification I and one or more stabilizer in a ratio of 8:1 to 1:1 w/w, expressed as d50 and measured either with DLS or with SLS, remains at 300 nm or less at storage for

at least one week at 40°C.

According to an embodiment of the present invention, the average particle size of the stable nanosuspension comprising nanoparticles of the compound of formula (I) in crystalline form of modification I and one or more stabilizer in a ratio of 8:1 to 1:1 w/w, expressed as d50 and measured either with DLS or with SLS, remains at 300 nm or less at storage for at least two weeks at 40°C.

According to an embodiment of the present invention, the average particle size of the stable nanosuspension comprising nanoparticles of the compound of formula (I) in crystalline form of modification I and one or more stabilizer in a ratio of 8:1 to 1:1 w/w, expressed as d50 and measured either with DLS or with SLS, remains at 300 nm or less at storage for at least four weeks at 40°C.

According to an embodiment of the present invention, the average particle size of the stable nanosuspension comprising nanoparticles of the compound of formula (I) in crystalline form of modification I and one or more stabilizer in a ratio of 8:1 to 1:1 w/w, expressed as d50 and measured either with DLS or with SLS, remains at 300 nm or less at storage for at least eight weeks at 40°C.

According to an embodiment of the present invention, the average particle size of the stable nanosuspension comprising nanoparticles of the compound of formula (I) in crystalline form of modification I and one or more stabilizer in a ratio of 8:1 to 1:1 w/w, expressed as d50 and measured either with DLS or with SLS, remains at 300 nm or less at storage for at least thirteen weeks at 40°C. One embodiment of the present invention is a stable nanosuspension comprising nanoparticles of methyl {4,6-diamino-2-[5-fluoro-1-(2-fluorobenzyl)-1H-pyrazolo[3,4-b]pyridin-3-yl]pyrimidin-5-yl}carbamate of the formula (I)

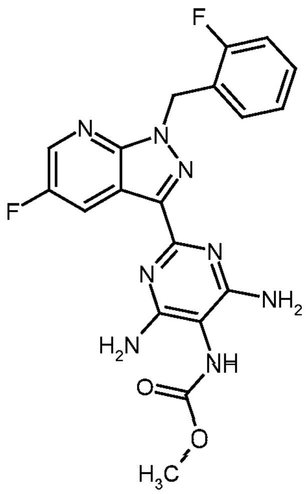

(I).

in crystalline form of modification I, characterized in that the x-ray diffractogram of the compound exhibits peak maxima of the 2 theta angle at 5.9, 6.9, 22.7, and one or more stabilizer(s) in a dispersing agent, wherein the one or more stabilizer is polyvinylpyrrolidone (PVP) in combination with sodium dodecylsulfate (SDS), or selected from the group consisting of vinylpyrrolidone-vinyl acetate copolymer, ethylene oxide-propylene oxide block copolymer, sodium dodecylsulfate (SDS), hydroxypropylmethylcellulose (HPMC) , polysorbate, hydroxypropylcellulose (HPC), polyoxyl-35 castor oil, polyoxyl 15 hydroxystearate, Na-desoxycholate, and combinations thereof, the maximum concentration of the one or more stabilizer(s) is the solubility limit of the stabilizer(s) in the suspension, the dispersing agent is selected from the group consisting of water, primary, secondary, and tertiary alcohols, and polyvalent alcohols, the nanoparticles have an average particle size, expressed as d50, of 500 nm or less, and the average particle size, expressed as d50, remains at 500 nm or less at storage for at least one week at a temperature of at least 40°C.

One embodiment of the present invention is a stable nanosuspension according to the invention, wherein the one or more stabilizer is selected from the group consisting of sodium dodecylsulfate, polyvinylpyrrolidone K10 to K50 in combination with sodium dodecylsulfate (SDS), hydroxypropylmethylcellulose, polysorbate 20-80, and combinations thereof.

One embodiment of the present invention is a stable nanosuspension comprising nanoparticles of methyl {4,6-diamino-2-[5-fluoro-1-(2-fluorobenzyl)-1H-pyrazolo[3,4-b]pyridin-3-yl]pyrimidin-5-yl}carbamate of the formula (I) in crystalline form of modification I, characterized in that the x-ray diffractogram of the compound exhibits peak maxima of the 2 theta angle at 5.9, 6.9, 22.7, and one or more stabilizer(s) in a dispersing agent, wherein the one or more stabilizer is selected from the group consisting of sodium dodecylsulfate, polyvinylpyrrolidone K10 to K50 in combination with sodium dodecylsulfate (SDS), hydroxypropylmethylcellulose, polysorbate 20-80, and combinations thereof, the maximum concentration of the one or more stabilizer(s) is the solubility limit of the stabilizer(s) in the suspension, the dispersing

agent is selected from the group consisting of water, primary, secondary, and tertiary alcohols, and polyvalent alcohols, the nanoparticles have an average particle size, expressed as d50, of 500 nm or less, and the average particle size, expressed as d50, remains at 500 nm or less at storage for at least one week at a temperature of at least 40°C.

One embodiment of the present invention is a stable nanosuspension according to the invention, wherein the ratio of the compound of formula (I):one or more stabilizer(s) is 16:1 to 1:2 w/w. One embodiment of the present invention is a stable nanosuspension according to the invention, wherein the ratio of the compound of formula (I):one or more stabilizer(s) is 8:1 to 2:1 w/w. In the case that a combination of two stabilizers is used and these two stabilizers are a surfactant and a polymer (e.g. SDS plus polyvinylpyrrolidone or SDS plus ethylene oxide-propylene oxide block copolymer), the ratio of compound (I):polymer is 16:1 to 1:2 w/w or 8:1 to 2:1 w/w and the concentration of the surfactant is 0.1-0.2% w/v.

One embodiment of the present invention is a stable nanosuspension comprising nanoparticles of methyl {4,6-diamino-2-[5-fluoro-1-(2-fluorobenzyl)-1H-pyrazolo[3,4-b]pyridin-3-yl]pyrimidin-5-yl}carbamate of the formula (I) in crystalline form of modification I, characterized in that the x-ray diffractogram of the compound exhibits peak maxima of the 2 theta angle at 5.9, 6.9, 22.7, and one or more stabilizer(s) in a dispersing agent, wherein the one or more stabilizer is selected from the group consisting of polyvinylpyrrolidone (PVP) in combination with sodium dodecylsulfate (SDS), vinylpyrrolidone-vinyl acetate copolymer, ethylene oxide-propylene oxide block copolymer, sodium dodecylsulfate (SDS), hydroxypropylmethylcellulose (HPMC) , polysorbate, hydroxypropylcellulose (HPC), polyoxyl-35 castor oil, polyoxyl 15 hydroxystearate, Na-desoxycholate, and combinations thereof, the maximum concentration of the one or more stabilizer(s) is the solubility limit of the stabilizer(s) in the suspension, the dispersing agent is selected from the group consisting of water, primary, secondary, and tertiary alcohols, and polyvalent alcohols, the nanoparticles have an average particle size, expressed as d50, of 500 nm or less, the average particle size, expressed as d50, remains at 500 nm or less at storage for at least one week at a temperature of at least 40°C, and wherein the ratio of the compound of formula (I):one or more stabilizer(s) is 16:1 to 1:2 w/w.

One embodiment of the present invention is a stable nanosuspension comprising nanoparticles of methyl {4,6-diamino-2-[5-fluoro-1-(2-fluorobenzyl)-1H-pyrazolo[3,4-b]pyridin-3-yl]pyrimidin-5-yl}carbamate of the formula (I) in crystalline form of modification I, characterized in that the x-ray diffractogram of the compound exhibits peak maxima of the 2 theta angle at 5.9, 6.9, 22.7, and one or more stabilizer(s) in a dispersing agent, wherein the one or more stabilizer is selected from the group consisting of polyvinylpyrrolidone (PVP) in combination with sodium dodecylsulfate (SDS), or ethylene oxide-propylene oxide block copolymer in combination with SDS, the maximum concentration of the one or more stabilizer(s) is the solubility limit of the stabilizer(s) in the suspension, the dispersing agent is selected from the group consisting of water, primary, secondary, and tertiary alcohols, and polyvalent alcohols, the nanoparticles have an average particle size, expressed as d50, of 500 nm or less, the average particle size, expressed as d50, remains at 500 nm or less at storage for at least one week at a temperature of at least 40°C, and wherein the ratio of the compound of formula (I):polyvinylpyrrolidone or ethylene oxide-propylene oxide block copolymer, respectively, is 16:1 to 1:2 w/w or 8:1 to 2:1 w/w and the concentration of SDS is 0.1-0.2% w/v.

One embodiment of the present invention is a stable nanosuspension according to the invention, wherein the nanoparticles have an average particle size, expressed as d50, of 500 nm or less, or 300 nm or less, or 250 nm or less or 200 nm or less, or 150 nm or less or 100 nm or less.

One embodiment of the present invention is a stable nanosuspension according to the invention, wherein the nanoparticles have an average particle size, expressed as d50, of 300 nm or less.

One embodiment of the present invention is a stable nanosuspension according to the invention, wherein the average particle size, expressed as d50, remains at 300 nm or less at storage for at least one week at a temperature of at least 40°C.

One embodiment of the present invention is a stable nanosuspension comprising nanoparticles of methyl {4,6-diamino-2-[5-fluoro-1-(2-fluorobenzyl)-1H-pyrazolo[3,4-b]pyridin-3-yl]pyrimidin-5-yl}carbamate of the formula (I) in crystalline form of modification I, characterized in that the x-ray diffractogram of the compound exhibits peak maxima of the 2 theta angle at 5.9, 6.9, 22.7, and one or more stabilizer(s) in a dispersing agent, wherein the one or more stabilizer is selected from the group consisting of polyvinylpyrrolidone (PVP) in combination with sodium dodecylsulfate (SDS), vinylpyrrolidone-vinyl acetate copolymer, ethylene oxide-propylene oxide block copolymer, sodium dodecylsulfate (SDS), hydroxypropylmethylcellulose (HPMC) , polysorbate, hydroxypropylcellulose (HPC), polyoxyl-35 castor oil, polyoxyl 15 hydroxystearate, Na-desoxycholate, and combinations thereof, the maximum concentration of the one or more stabilizer(s) is the solubility limit of the stabilizer(s) in the suspension, the dispersing agent is selected from the group consisting of water, primary, secondary, and tertiary alcohols, and polyvalent alcohols, the nanoparticles have an average particle size, expressed as d50, of 500 nm or less, and the average particle size, expressed as d50, remains at 500 nm or less, or 300 nm or less, or 250 nm or less or 200 nm or less, or 150 nm or less or 100 nm or less at storage for at least one week, or at least two weeks, or at least four weeks, or at least eight weeks, or at least thirteen weeks at a temperature of at least 40°C.

In order to obtain the stable nanosuspensions according to the present invention, specific stabilizer(s) and combinations of stabilizer(s) had to be identified (Fig. 1). This task is highly dependent on the specific compound. Fig. 2

shows the long-term stability of the nanosuspensions of the invention, which is a prerequisite for their use as such and for processing in further pharmaceutical forms, such as granules and tablets. Contrary to the teaching of the prior art that stabilizer concentrations above CMC promote Ostwald ripening, it was surprisingly found that stable nanosuspension could be obtained with concentrations of at least the stabilizer SDS as high as 4-fold above the CMC (Fig. 3, API:SDS = 2:1). As shown in Fig. 4, particle sizes after nanomilling of micronized vericiguat are significantly smaller than particle sizes of vericiguat that was only micronized.

In order to obtain stable nanosuspensions, the parameters of wet bead milling had to be carefully selected in addition to the choice and concentration of stabilizer(s).

One embodiment of the present invention is a process for preparing the stable nanosuspension according to the invention, comprising the steps of

a. suspending the compound of formula (I) in crystalline form of modification I in a dispersing agent and one or more stabilizer according to the invention, wherein the maximum concentration of the one or more stabilizer(s) is the solubility limit of the stabilizer(s) in the suspension;

b. wet bead milling the suspension generated in step a. with a specific energy input of 10,000 kJ/kg or more and a stress intensity of $0.004 \cdot 10^{-3}$ Nm to $1 \cdot 10^{-3}$ Nm.

One embodiment of the present invention is the process for preparing the stable nanosuspension according to any embodiment of the invention, wherein the dispersing agent is selected from the group consisting of water, primary, secondary, and tertiary alcohols, and polyvalent alcohols.

One embodiment of the present invention is the process for preparing the stable nanosuspension according to any embodiment of the invention, wherein the one or more stabilizer is selected from the group consisting of polyvinylpyrrolidone (PVP) in combination with sodium dodecylsulfate (SDS), vinylpyrrolidone-vinyl acetate copolymer, ethylene oxide-propylene oxide block copolymer, sodium dodecylsulfate (SDS), hydroxypropylmethylcellulose (HPMC), polysorbate, hydroxypropylcellulose (HPC), polyoxyl-35 castor oil, polyoxyl 15 hydroxystearate, Na-desoxycholate, and combinations thereof.

One embodiment of the present invention is the process for preparing the stable nanosuspension according to any embodiment of the invention, wherein the one or more stabilizer is selected from the group consisting of sodium dodecylsulfate, polyvinylpyrrolidone K10 to K50 in combination with sodium dodecylsulfate (SDS), hydroxypropylmethylcellulose, polysorbate 20-80, and combinations thereof.

One embodiment of the present invention is a process for preparing the stable nanosuspension according to the invention, comprising the steps of

a. suspending the compound of formula (I) in crystalline form of modification I in a dispersing agent selected from the group consisting of water, primary, secondary, and tertiary alcohols, and polyvalent alcohols and one or more stabilizer selected from the group consisting of polyvinylpyrrolidone (PVP) in combination with sodium dodecylsulfate (SDS), vinylpyrrolidone-vinyl acetate copolymer, ethylene oxide-propylene oxide block copolymer, sodium dodecylsulfate (SDS), hydroxypropylmethylcellulose (HPMC), polysorbate, hydroxypropylcellulose (HPC), polyoxyl-35 castor oil, polyoxyl 15 hydroxystearate, Na-desoxycholate, and combinations thereof, wherein the maximum concentration of the one or more stabilizer(s) is the solubility limit of the stabilizer(s) in the suspension;

b. wet bead milling the suspension generated in step a. with a specific energy input of 10,000 kJ/kg or more and a stress intensity of $0.004 \cdot 10^{-3}$ Nm to $1 \cdot 10^{-3}$ Nm.

Within the meaning of the present invention, "wet bead milling" is used synonymously to "wet ball milling", and also synonymously to "wet milling". The capacity and fineness can be adjusted by adjusting the diameter of the ball/bead.

Milling parameters including milling bead size, milling bead material, tip speed, duration of milling, and volumetric filling with milling beads were optimized to obtain the nanoformulations according to the invention.

Within the meaning of the present invention, the term "milling" is used synonymously with the term "grinding".

**In** order to obtain the nanoformulations according to the invention, sufficient specific energy input and stress intensity are needed. The specific energy input and/or stress intensity are sufficient if the desired particle size is reached at sufficient stabilization. If the particle size is too high, then either specific energy input and/or stress intensity and/or stabilization are not sufficient.

Within the meaning of the present invention, specific energy input is defined as the net energy input related to the product mass.

Within the meaning of the present invention, stress intensity is defined according to Kwade et al. (1996). According to Kwade, an optimum stress intensity exists for a fixed specific energy input, for which the finest product is

achieved. With increasing specific energy input, and therefore increasing product fineness, the optimum stress intensity decreases. For a constant specific energy input the stress intensity determines the product fineness.

Within the meaning of the present invention "wet bead milling" or "nanomilling" is defined as the preparation of drug nanoparticles via wet media milling (nanomilling). As is known to the person skilled in the art, milling beads are used in "wet bead milling" or "nanomilling". The basic principle behind nanomilling is increasing the surface area-to-volume ratio of an API by reducing the particle size measured as d90 below 800 nm, typically in the hundred to several hundreds of nm range, measured as d50. This conversion of drug particles into nanocrystals allows for greater interaction with water, which increases dissolution rate. In general terms, smaller particles dissolve more quickly.

According to an embodiment of the present invention, wet bead milling of the suspension is carried out in the presence of milling beads having a size of 0.05-0.8 mm. According to an embodiment of the present invention, the milling beads are made from a material selected from the group consisting of ceramics, glass, polymers, and steel. Within the meaning of the present invention, examples for ceramics include yttrium-stabilized zirconium oxide, cerium-stabilized zirconium oxide, and zirconium silicate, e.g. sintered or fused. Examples for glass include glass doped with zirconia. Examples for polymers include polystyrol. According to an embodiment of the present invention, the milling beads are made from yttrium-stabilized zirconium oxide. According to an embodiment of the present invention, the volumetric filling with milling beads is 40-85% v/v. According to an embodiment of the present invention, the volumetric filling with milling beads is 50-85% v/v. According to an embodiment of the present invention, the volumetric filling with milling beads is 60-85% v/v. According to an embodiment of the present invention, the volumetric filling with milling beads is 60-80% v/v.

One embodiment of the present invention is the process for preparing the stable nanosuspension according to any embodiment of the invention, wherein step b. is carried out in the presence of milling beads having a size of 0.05-0.8 mm.

One embodiment of the present invention is the process for preparing the stable nanosuspension according to any embodiment of the invention, wherein step b. is carried out in the presence of milling beads made from a material selected from the group selected from ceramics, glass, polymers, and steel.

One embodiment of the present invention is the process for preparing the stable nanosuspension according to any embodiment of the invention, wherein the volumetric filling with beads in step b. is 50-85% v/v.

The specific energy input of 10,000 kJ/kg or more and the stress intensity of $0.004 \cdot 10^{-3}$ Nm to $1 \cdot 10^{-3}$ Nm are related e.g. to the tip speed and the duration of milling.

According to one embodiment of the present invention, examples for the tip speed are 8-15 m/s or 12-15 m/s, and the duration of milling is 10 min to 12 h, provided that the specific energy input is 10,000 kJ/kg or more and the stress intensity of $0.004 \cdot 10^{-3}$ Nm to $1 \cdot 10^{-3}$ Nm. According to one embodiment of the present invention, examples for the tip speed are 8-15 m/s and the duration of milling is 30 min to 8 h, provided that the specific energy input is 10,000 kJ/kg or more and the stress intensity of $0.004 \cdot 10^{-3}$ Nm to $1 \cdot 10^{-3}$ Nm. According to one embodiment of the present invention, examples for the tip speed are 8-15 m/s, and the duration of milling is 30 min to 6 h, provided that the specific energy input is 10,000 kJ/kg or more and the stress intensity of $0.004 \cdot 10^{-3}$ Nm to $1 \cdot 10^{-3}$ Nm. According to one embodiment of the present invention, examples for the tip speed are 8-15 m/s, and the duration of milling is 30 min to 2 h, provided that the specific energy input is 10,000 kJ/kg or more and the stress intensity of $0.004 \cdot 10^{-3}$ Nm to $1 \cdot 10^{-3}$ Nm.

According to one embodiment of the present invention, the milling beads are made from yttrium-stabilized zirconium oxide, the milling beads have a size of 0.05-0.6 mm, the volumetric filling with milling beads is 60-80%, the tip speed is 12-15 m/s and the duration of milling is 60 min.

One embodiment of the present invention is the process for preparing the stable nanosuspension according to any embodiment of the invention, wherein step b. is carried out in the presence of milling beads having a size of 0.05-0.8 mm, made from a material selected from the group consisting of ceramics, glass, polymers, and steel, and wherein the volumetric filling with beads in step b. is 50-85% v/v.

One embodiment of the present invention involves a further step before wet bead nanomilling that is performed in order to destroy coarse particles and improve the result of the subsequent nanomilling step. Coarse particles may be destroyed e.g. by micronization or by wet bead milling using beads having the size of 1-2 mm.

One embodiment of the present invention is the process for preparing the stable nanosuspension according to any embodiment of the invention, wherein in step a., the compound of formula (I) in crystalline form of modification I is micronized in a first step before suspending it in a dispersing agent and one or more stabilizer.

One embodiment of the present invention is the process for preparing the stable nanosuspension according to any embodiment of the invention, wherein in step a., the compound of formula (I) in crystalline form of modification I is milled in a first wet bead milling step in the presence of milling beads having a size of 1-2 mm, before suspending it in a dispersing agent and one or more stabilizer.

One embodiment of the present invention is the process for preparing the stable nanosuspension according to any embodiment of the invention, comprising the steps of

a. micronizing the compound of formula (I) in crystalline form of modification I in a first step before suspending it in a

dispersing agent selected from the group consisting of water, primary, secondary, and tertiary alcohols, and polyvalent alcohols, and one or more stabilizer selected from the group consisting of polyvinylpyrrolidone (PVP) in combination with sodium dodecylsulfate (SDS), vinylpyrrolidone-vinyl acetate copolymer, ethylene oxide-propylene oxide block copolymer, sodium dodecylsulfate (SDS), hydroxypropylmethylcellulose (HPMC), polysorbate, hydroxypropylcellulose (HPC), polyoxyl-35 castor oil, polyoxyl 15 hydroxystearate, Na-desoxycholate, and combinations thereof, wherein the maximum concentration of the one or more stabilizer(s) is the solubility limit of the stabilizer(s) in the suspension;

b. wet bead milling the suspension generated in step a. with a specific energy input of 10,000 kJ/kg or more and a stress intensity of 0.004 • 10-3 Nm to 1 • 10-3 Nm, wherein the milling beads used have a size of 0.05-0.8 mm.

One embodiment of the present invention is the process for preparing the stable nanosuspension according to any embodiment of the invention, comprising the steps of

a. milling the compound of formula (I) in crystalline form of modification I in a first wet bead milling step in the presence of milling beads having a size of 1-2 mm before suspending it in a dispersing agent selected from the group consisting of water, primary, secondary, and tertiary alcohols, and polyvalent alcohols, and one or more stabilizer selected from the group consisting of polyvinylpyrrolidone (PVP) in combination with sodium dodecylsulfate (SDS), vinylpyrrolidone-vinyl acetate copolymer, ethylene oxide-propylene oxide block copolymer, sodium dodecylsulfate (SDS), hydroxypropylmethylcellulose (HPMC), polysorbate, hydroxypropylcellulose (HPC), polyoxyl-35 castor oil, polyoxyl 15 hydroxystearate, Na-desoxycholate, and combinations thereof, wherein the maximum concentration of the one or more stabilizer(s) is the solubility limit of the stabilizer(s) in the suspension;
b. wet bead milling the suspension generated in step a. with a specific energy input of 10,000 kJ/kg or more and a stress intensity of 0.004 • 10-3 Nm to 1 • 10-3 Nm, wherein the milling beads used have a size of 0.05-0.8 mm.

One embodiment of the present invention is a process for preparing the stable nanosuspension according to the invention, comprising the steps of

a. suspending the compound of formula (I) in crystalline form of modification I in a dispersing agent and one or more stabilizer according to the invention, wherein the maximum concentration of the one or more stabilizer(s) is the solubility limit of the stabilizer(s) in the suspension;

b. wet bead milling the suspension generated in step a. with a specific energy input of 15,000 kJ/kg or more, or 20,000 kJ/kg or more, or 25,000 kJ/kg or more, and a stress intensity of 0.004 • $10^{-3}$ Nm to 1 • $10^{-3}$ Nm.

One embodiment of the present invention is a process for preparing the stable nanosuspension according the invention, comprising the steps of

a. suspending the compound of formula (I) in crystalline form of modification I in a dispersing agent selected from the group consisting of water, primary, secondary, and tertiary alcohols, and polyvalent alcohols, and one or more stabilizer selected from the group consisting of polyvinylpyrrolidone (PVP) in combination with sodium dodecylsulfate (SDS), vinylpyrrolidone-vinyl acetate copolymer, ethylene oxide-propylene oxide block copolymer, sodium dodecylsulfate (SDS), hydroxypropylmethylcellulose (HPMC), polysorbate, hydroxypropylcellulose (HPC), polyoxyl-35 castor oil, polyoxyl 15 hydroxystearate, Na-desoxycholate, and combinations thereof, wherein the maximum concentration of the one or more stabilizer(s) is the solubility limit of the stabilizer(s) in the suspension;

b. wet bead milling the suspension generated in step a. with a specific energy input of 10,000 kJ/kg or more, or 15,000 kJ/kg or more, or 20,000 kJ/kg or more, or 25,000 kJ/kg or more, and a stress intensity of 0.004 • 10-3 Nm to 1 • 10-3 Nm, wherein the milling beads used have a size of 0.05-0.8 mm.

It is known that decreasing the particle size of an API might lead to an increased dissolution velocity and thus can lead to a higher bioavailability and potentially influence the extent of a food effect. However, the selection of a process for reduction of the particle size, selection of parameters for process-equipment, namely for preparation of stable nanosuspensions and stable nanoparticles, as well as the selection of optimal stabilizers for a drug nanosuspension are challenging and are not obvious in view of the prior art.

The upper limit of the specific energy input is related to the degree of amorphization of the compound of formula (I) caused by the milling process. Complete amorphization is to be avoided since in that case the crystal structure of the compound of formula (I) is lost in its entirety and recrystallization occurs in an uncontrolled way. As shown in Fig. 5a to c,

surprisingly no amorphization of the compound (I) in crystalline modification I occurs during the wet bead nanomilling process.

The choice of the mill is decisive for producing nanoparticles. Nanomilling has to be performed as wet bead milling, for example in a planetary mill or stirred media mill. In contrast, jet mills - another type of mills used e.g. for micronization - perform dry milling. Jet mills are suitable for grinding of particles down to the micrometer range. The grinding action in a jet mill is created by the high-velocity collisions between particles driven by multiple jets of air or steam. Dry milling is not suitable for producing nanoparticles.

Subject of the disclosure are particles comprising the compound of formula (I) in crystalline form of modification I and one or more stabilizer(s) in a ratio of the compound of formula (I):one or more stabilizer(s) of 16:1 to 1:2 w/w, wherein said particles have an average particle size expressed as d50 of 500 nm or less in dried form.

One embodiment of the invention are dried nanoparticles comprising the compound of formula (I) in crystalline form of modification I and one or more stabilizer(s) in a ratio of the compound of formula (I):one or more stabilizer(s) of 16:1 to 1:2 w/w, wherein the nanoparticles have an average particle size expressed as d50 of 500 nm or less.

One embodiment of the invention are dried nanoparticles comprising the compound of formula (I) in crystalline form of modification I and a combination of two stabilizers, PVP and SDS, in a ratio of the compound of formula (I):PVP of 16:1 to 1:2 w/w, and a concentration of SDS of 0.1-0.2% w/v, wherein the nanoparticles have an average particle size expressed as d50 of 500 nm or less.

One embodiment of the invention are dried nanoparticles comprising the compound of formula (I) in crystalline form of modification I and a combination of two stabilizers, ethylene oxide-propylene oxide block copolymer and SDS, in a ratio of the compound of formula (I): ethylene oxide-propylene oxide block copolymer of 16:1 to 1:2 w/w, and a concentration of SDS of 0.1-0.2% w/v, wherein the nanoparticles have an average particle size expressed as d50 of 500 nm or less.

Within the meaning of the present invention, the terms "dried" or "in dried form", used for example in the terms "dried nanoformulations" or "(nano)particles in dried form" or "(nano)particles having an average particle size expressed as d50 of 500 nm or less in dried form", are defined as nanosuspensions according to the invention that have been transferred to the solid state by a drying step. Dried nanoformulations or nanoparticles still contain one or more stabilizer(s) but do not contain a dispersing agent anymore. The dispersing agent is removed by performing a drying step.

Subject of the disclosure is a pharmaceutical composition in solid form made with the nanosuspension of the present invention in dried form, which is manufactured by pharmaceutical processes, optionally including granulation, and including compression, and coating.

Subject of the disclosure is a pharmaceutical composition in solid form made with the nanosuspension of the present invention in dried form which is manufactured by pharmaceutical processes, including granulation, compression, and coating.

According to one embodiment of the invention, the drying of the nanosuspension according to the invention can be carried out by spray drying or with concomitant granulation by fluidized bed granulation.

The production of the solid dosage forms may be carried out via a wet granulation process (high shear granulation or fluidized bed granulation). High shear granulation is a shaping process for wet granulation. A binder liquid is fed to the powder particles in a closed container with blending tools and a chopper. Dense granules are formed through the liquid and solid bridges that result. Fluidized bed granulation is also a wet granulation process that involves the addition of a binder liquid to primary particles to form aggregated granulates. While the binder liquid is sprayed from the top, the particles are fluidized from the bottom.

According to one embodiment of the present invention, top spray granulation is used in fluidized bed granulation.

The tabletting is preferably carried out with the initially produced granulate. This may be followed by a coating of the solid dosage forms.

In wet granulation the active compound product is suspended in the granulating liquid. The employed granulating liquid contains a solvent, a hydrophilic binder and a wetting agent. The hydrophilic binder is dispersed in the granulating fluid or preferably dissolved therein. Employable solvents for the granulating liquid include organic solvents, for example ethanol or acetone or water or mixtures thereof. It is preferable when water is used as solvent. Hydrophilic binders employed are pharmaceutically acceptable hydrophilic additives, preferably those which dissolve in the solvent of the granulating fluid. Preferably employed here are hydrophilic polymers such as for example hydroxypropylmethylcellulose (HPMC), sodium carboxymethylcellulose, methylcellulose, hydroxypropylcellulose (HPC), low-substituted hydroxypropylcellulose (L-HPC), hydroxypropylcellulose LF, polyvinylpyrrolidone, polyvinyl alcohol, vinylpyrrolidone-vinyl acetate copolymers (for example Kollidon® VA64, BASF), gelatin, guar gum, partially hydrolyzed starch, alginates or xanthan. It is particularly preferable to use hydroxypropylmethylcellulose (HPMC) as a hydrophilic binder. The hydrophilic binder is present at a concentration of 1% to 12% (based on the total mass of the pharmaceutical dosage form), preferably 1% to 6%. Wetting agents employed are pharmaceutically acceptable compounds as sodium laurilsulfate, polysorbate, polyethylene glycol (15)-hydroxystearate, or polyethylene glycol hexadecyl ether.

The premix of the wet granulation contains further pharmaceutically acceptable additives, such as for example

fillers, binders and disintegration promoters (disintegrants). Fillers and binders are for example cellulose powder, microcrystalline cellulose, silicified microcrystalline cellulose, hydroxypropylcellulose, lactose monohydrate, mannitol, maltitol, sorbitol and xylitol, preferably microcrystalline cellulose or mannitol or a mixture of microcrystalline cellulose and mannitol/lactose monohydrate. Disintegration promoters (disintegrants) are for example carboxymethylcellulose, croscarmellose (crosslinked carboxymethylcellulose), crospovidone (crosslinked polyvinylpyrrolidone), low-substituted hydroxypropylcellulose (L-HPC), sodium carboxymethyl starch, potato sodium starch glycolate, partially hydrolyzed starch, wheat starch, maize starch, rice starch and potato starch.

The obtained granulate is subsequently converted into solid dosage forms. Pharmaceutically acceptable additives added are, for example, lubricants, glidants, flow regulators and disintegration promoters (disintegrants). Lubricants, glidants, flow regulators are for example fumaric acid, stearic acid, sodium stearyl fumarate, magnesium stearate, higher molecular weight fatty alcohols, starches (wheat, rice, maize or potato starch), talc, high-dispersity (colloidal) silicon dioxide and glycerol distearate. Disintegration promoters (disintegrants) are for example carboxymethylcellulose, croscarmellose (crosslinked carboxymethylcellulose), crospovidone (crosslinked polyvinylpyrrolidone), low-substituted hydroxypropylcellulose (L-HPC), sodium carboxymethyl starch, partially hydrolyzed starch, wheat starch, maize starch, rice starch and potato starch.

Solid dosage forms are optionally coated under customary conditions familiar to those skilled in the art in a further step. The coating is effected by addition of coating and film-forming agents such as hydroxypropylcellulose, hydroxypropylmethylcellulose (for example hydroxypropylmethylcellulose 5cP or 15 cP), polyvinylpyrrolidone, vinylpyrrolidone-vinyl acetate copolymers (for example Kollidon® VA64, BASF), shellac, glyceryl triacetate, triethyl citrate, talc as an antiadhesive agent and/or colourants/pigments such as titanium dioxide, iron oxides, indigotin or suitable coloured coatings.

One embodiment of the present invention comprises solid dosage forms containing active compound product of the compound of formula (I) in the form of nanoparticles and further containing microcrystalline cellulose, lactose monohydrate, hydroxypropylmethylcellulose 3cP and/or 5cP, sodium lauryl sulfate or polysorbate 20, sodium croscarmellose, magnesium stearate, talc, iron oxides and titanium dioxide. The nanoparticles still contain one or more stabilizer(s) but the dispersing agent was removed by drying.

A further embodiment of the present invention comprises solid dosage forms containing active compound product of the compound of formula (I) produced by a process for formation of nanoparticles according to the invention, wherein the solid dosage forms contain 1.25 to 20 mg of the active compound product of the compound of formula (I) per solid dosage form. Further embodiments comprise solid dosage forms containing 1.25 mg, 2.5 mg, 5.0 mg, 7.5 mg, 10 mg, 12.5 mg, 15 mg, 17.5 mg or 20 mg of active compound product of the compound of formula (I) produced by a process according to the invention per solid dosage form.

One embodiment of the present invention are dried nanoparticles comprising the methyl {4,6-diamino-2-[5-fluoro-1-(2-fluorobenzyl)-1H-pyrazolo[3,4-b]pyridin-3-yl]pyrimidin-5-yl}carbamate compound of the formula (I) in crystalline form of modification I, characterized in that the x-ray diffractogram of the compound exhibits peak maxima of the 2 theta angle at 5.9, 6.9, 22.7, and one or more stabilizer(s) in a ratio of the compound of formula (I):one or more stabilizer(s) of 16:1 to 1:2 w/w, wherein the dried nanoparticles have an average particle size expressed as d50 of 500 nm or less.

One embodiment of the present invention is a pharmaceutical composition in solid form comprising dried nanoparticles of methyl {4,6-diamino-2-[5-fluoro-1-(2-fluorobenzyl)-1H-pyrazolo[3,4-b]pyridin-3-yl]pyrimidin-5-yl}carbamate of the formula (I) in crystalline form of modification I, characterized in that the x-ray diffractogram of the compound exhibits peak maxima of the 2 theta angle at 5.9, 6.9, 22.7, and one or more stabilizer(s) having an average particle size, expressed as d50, of 500 nm or less, made with the nanoparticles according to the invention.

One embodiment of the present invention is a pharmaceutical composition according to the invention, wherein the solid form is selected from the group consisting of granules and tablets.

One embodiment of the present invention are dried nanoparticles or a pharmaceutical composition according to the invention, wherein the dried nanoparticles or the pharmaceutical composition do not contain a dispersing agent.

Apart from solubility in water / biorelevant media, stabilizers in nanoformulations have to fulfil additional important properties. They need to ensure short-term stabilization of the nanosuspension against (re)agglomeration. This property is inevitable for a nanoformulation process and is the basic requirement to produce nanoparticles in a milling process. Further, stabilizers need to ensure long-term stabilization of the nanosuspension. Once the nanosuspension is produced, this suspension has to be stable for a distinct time span until drying can be performed. Within that time re-agglomeration, crystal growth (Ostwald ripening) and sedimentation have to be inhibited by appropriate selection of stabilizers. In addition, it has to be ensured that nano-size particles are still present after reconstitution of dried material. During spray drying an almost instantaneous transition of the nanosuspension into solid particles occurs. That phase change implicates the potential for changes of the nanoparticles of an active compound, including melting, agglomeration, or dissolution in polymer. By identifying appropriate spray drying parameters and suitable excipients, it has to be ensured that the particles of the active compound remain separated nanoparticles after dissolution of the dried material.

An additional hurdle and challenge is the further processing of a nanosuspension containing methyl {4,6-diamino-2-[5-fluoro-1-(2-fluorobenzyl)-1H-pyrazolo[3,4-b]pyridin-3-yl]pyrimidin-5-yl}carbamate (Vericiguat, compound of formula (I)) to granules and tablets as final dosage form. Potential particle size increase or particle growth can also occur during subsequent processing, such as granulation and compression, and can lead to loss of high surface area associated with the drug nanoparticles, which reduces the significant benefits intended from the nanomilling process.

As shown in Fig. 6-11, the stable nanosuspensions according to the invention and the dried pharmaceutical forms made from them led to an increased drug release. This was observed for nanosuspensions as well as granules and tablets made from them. Increased drug release was observed not only in 0.01N HCl, but also in bioequivalent medium. (FeSSIF) Confocal laser scanning microscopy (CLSM) pictures of granules containing only micronized (Fig 12a) compound of the formula (I) clearly showed light spots representing fluorescent compound of the formula (I). In contrast, as shown in Fig. 12b, CLSM pictures of micronized and nanomilled compound of the formula (I) clearly shows a much smoother surface with no light spots. These pictures are clearly indicative of preserved nanoparticles following micronization and nanomilling.

Finally, it must be ensured that nanoparticles are retained when in solid formulations made from nanosuspensions and that nanoparticles are also still present at the time of resuspension at the site of absorption. As shown in Tables 7 and 8, nano-size particles are still present after reconstitution of dried material that was obtained by drying of nanosuspensions, depending on the nature and amount excipients/stabilizers used.

The fact that it was possible to transfer the nanosuspensions to the solid state and at the same time maintain the superior bioavailability of the dried nanosuspensions, was a surprising effect over the prior art.

One embodiment of the present invention are stable nanosuspensions comprising the compound of formula (I) in crystalline form of modification I and one or more stabilizer(s) in a dispersing agent according to the invention for use in the treatment and/or prophylaxis of heart failure, worsening chronic heart failure, worsening chronic heart failure with reduced ejection fraction, worsening chronic heart failure with preserved ejection fraction, angina pectoris, hypertension, pulmonary hypertension, ischaemias, vascular disorders, cognitive disorders, kidney failure, thromboembolic disorders, fibrotic disorders and arteriosclerosis.

One embodiment of the present invention are dried nanoparticles comprising the compound of formula (I) in crystalline form of modification I and one or more stabilizer(s) according to the invention for use in the treatment and/or prophylaxis of heart failure, worsening chronic heart failure, worsening chronic heart failure with reduced ejection fraction, worsening chronic heart failure with preserved ejection fraction, angina pectoris, hypertension, pulmonary hypertension, ischaemias, vascular disorders, cognitive disorders, kidney failure, thromboembolic disorders, fibrotic disorders and arteriosclerosis.

One embodiment of the present invention is a pharmaceutical composition in solid form comprising dried nanoparticles of the compound of formula (I) in crystalline form of modification I and one or more stabilizer(s) in a dispersing agent according to the invention for use in the treatment and/or prophylaxis of heart failure, worsening chronic heart failure, worsening chronic heart failure with reduced ejection fraction, worsening chronic heart failure with preserved ejection fraction, angina pectoris, hypertension, pulmonary hypertension, ischaemias, vascular disorders, cognitive disorders, kidney failure, thromboembolic disorders, fibrotic disorders and arteriosclerosis.

One embodiment of the present invention is a method for the treatment and/or prophylaxis of heart failure, worsening chronic heart failure, worsening chronic heart failure with reduced ejection fraction, worsening chronic heart failure with preserved ejection fraction, angina pectoris, hypertension, pulmonary hypertension, ischaemias, vascular disorders, cognitive disorders, kidney failure, thromboembolic disorders, fibrotic disorders and arteriosclerosis in humans and animals by administration of an effective amount of a pharmaceutical composition in solid form made with the nanosuspensions comprising the compound of formula (I) in crystalline form of modification I and one or more stabilizer(s) in a dispersing agent according to the invention.

An improved bioavailability of this nanosuspension was shown in a rat study in comparison to a tylose suspension with only micronized material of the compound of formula (I). The effect of the nano scale on an improved bioavailability could be maintained even after drying of the suspension within a fluidized bed granulator which is a very sensitive production step prone to API particle agglomeration or Ostwald ripening and thus the risk of losing the benefits of the nanoparticles. Studies of the relative bioavailability were performed in rats with granules and mini-tablets. The formulations with nanosized starting material for processing resulted in higher bioavailability in vitro and in vivo than with micronized starting material, as shown in Example 5, Table 3, Example 6, Tables 5 to 7, and Fig. 6-11.

Due to the dissolution-limited behaviour of the compound of formula (I) (Vericiguat), bioavailability in preclinical and clinical studies decreased with higher doses. With a nanoformulation, bioavailability limited by dissolution velocity can be increased in general and a food effect may be circumvented.

The shown data is surprising over the state of the art because achieving the development of a stable nanosuspension using SDS as stabilizer could not be anticipated. Further, it was surprising that the selection of stabilizer was a crucial process parameter to produce nanoparticles via wet bead milling. The superiority of using SDS instead of Vitamin E TPGS as stabilizer that is shown by smaller particle sizes and the lack of amorphization after milling, both leading to smaller nanoparticles as important parameter for increasing bioavailability, was also surprising over the state of the art.

The ratio of compound (I) : SDS used in the experiments was 2:1. The content of compound (I) was 2%, thus the content of SDS was 1%. The critical micelle concentration (CMC) of SDS is 0.23% or 8.2 mM. The concentration of SDS used in the experiments was thus more than 4-times of its CMC. The concentration of surfactant used, e.g. SDS, to achieve a stable nanoformulation according to the invention is thus clearly above the critical micelle concentration (CMC). As outlined in the prior art, a concentration of surfactant clearly above CMC is considered to accelerate Ostwald ripening. It was thus surprising that in the present invention, no Ostwald ripening was observed.

The nanoparticles were physically stabilized with sodium dodecyl sulfate (SDS) in a concentration range of API/SDS from 8:1 to 2:1 w/w. Surprisingly, nanoparticles stabilized with Vitamin E TPGS, known from prior art, showed inferior results with respect to higher particle sizes and inferior bioavailability in comparison to similarly produced nanoparticles with SDS as stabilizer, as shown in Fig. 1, and Example 5, Table 3, Example 6, Tables 4-6, and Fig. 10.

In a first step, a broad screening of stabilizers revealed that not all excipients described in literature are suitable for producing nanosuspensions of this compound. Human serum albumin, egg lecithin, hydroxypropylmethylcellulose acetate succinate (HPMC-AS) or sodium oleate resulted in unfavourable products with either too large particle or agglomeration phenomena.

A beneficial effect of stable nanosuspensions and nanoparticles according to the invention is an increased bioavailability of the compound of formula (I).

## Examples

### Abbreviations

| | |
|---|---|
| AcDiSol | croscarmellose sodium |
| API | active pharmaceutical ingredient |
| Avicel PH 101 | cellulose microcrystalline |
| BKC | Benzalkonium chloride |
| CMC | critical micelle concentration |
| Compound (I) | Compound of formula (I), vericiguat |
| Cremophor® EL | Polyoxyl-35 castor oil, also known as Kolliphor® EL, CAS number 61791-12-6 |
| DLS | dynamic light scattering (via photon correlation spectroscopy) |
| Dn(50) | particle size 50 measured by dynamic light scattering |
| DOSS | Dioctyl Sulfosuccinate |
| Eudragit® E PO | Poly(butyl methacrylate-co-(2- dimethylaminoeethyl) methacrylate-co-methyl methacrylate) in a ratio of 1:2:1 |
| FaSSIF | fasted state simulated intestinal fluid |
| FBG | fluidized bed granulation |
| FeSSIF | fed state simulated intestinal fluid |
| HPC | Hydroxypropylcellulose, CAS number 9004-64-2 |
| HPMC 5cP | Hydroxypropylmethylcellulose 5 cP. A 2% aqueous solution of HPMC 5cP has a viscosity of 5 mPas at 20°C |
| HPMC 3cP | Hydroxypropylmethylcellulose 3 cP. A 2% aqueous solution of HPMC 3cP has a viscosity of 2.4-3.6 mPas at 20°C |
| HPMC AS | Hydroxypropylmethylcellulose acetate succinate |
| HPC SSL | Hydroxypropylcellulose SSL. A 2% aqueous solution of HPC SSL has a viscosity of 2-2.9 mPas at 20°C |
| logP | Logarithm of the partition coefficient |
| micron. | Micronized |
| o. | Oval |

(continued)

| | |
|---|---|
| PDI | polydispersity index = heterogeneity index |
| PBM | Planetary ball mill |
| PVP K12/K17/K30 | Polyvinylpyrrolidone (Povidone), CAS number: 9003-39-8 with average molecular weight of 12,000; 17,000; and 30,000 respectively |
| PVP VA 64 | Polyvinylpyrrolidone-vinyl acetate copolymer, CAS number 25086-89-9, Kollidon® VA 64 |
| r. | Round |
| rh | relative humidity |
| SDS | sodium dodecyl sulfate |
| SLS | Static light scattering (via laser diffraction) |
| Solutol® HS 15 | also: Kolliphor® HS 15, polyoxyl 15 hydroxystearate, CAS number 70142-34-6 |
| USP | United States Pharmacopeia |
| Vitamin E TPGS | d-alpha tocopheryl polyethylene glycol 1000 succinate, CAS number 9002-96-4 |
| $ZrO_2$ | Zirkonium oxide |

## Wet bead milling equipment

### Method A: Planetary mill

Most of the screening experiments of Example 1 were done in a planetary ball mill (PBM) of type Pulverisette 5. For sample preparation, the micronized compound of the formula (I) was predispersed in aqueous polymer surfactant solution and transferred into the grinding chamber (PET 23ml) which was filled with milling beads. The water contained in the solution was used as dispersing agent. As grinding media (0.4 - 0.6 mm) the Silibeads® zirconium oxide (yttrium stabilized) with a bulk density of 3.9 kg/l from Sigmund Lindner (Germany) were used. The milling beads fill level was 60% of the grinding chamber volume. Care was taken not to leave any air inclusions in the grinding chamber, as this would have a negative effect on the grinding process. The grinding time was 90 minutes at 400 rotations per minute. Four grinding chambers could be fed into the PBM per grinding operation. Thus, after separation of the grinding media, about 40 - 45 g suspension per grinding process could be produced.

### Method B: Stirred media mill

For producing higher amounts of nanosuspension (e.g. for animal tests) a stirred media mill was used either the picoliq (Hosokawa Alpine) or Netzsch Labstar.

The drug nanosuspension was produced by wet grinding micronized compound of the formula (I) in a stirred media mill of the type Hosokawa Picoliq with 19 ml grinding chamber volume. Grinding was performed with an agitator. The rotational speed was 10000 rpm. The grinding media were identical to those in the PBM, including the use of water as dispersing agent. The grinding media fill level was 80% of the grinding chamber volume. The mill was operated in batch mode. 13.6 g of suspension were ground. The entire system was cooled by a cryostat so that a maximum of 29°C was measured at the outlet of the grinding chamber during milling.

Alternatively, the drug nanosuspension was produced by wet grinding micronized compound of the formula (I) in a stirred media mill of the type Netzsch LabStar with 120ml grinding chamber volume. Grinding was performed with a disk agitator. The rotational speed was 2770 rpm. The grinding media were identical to those in the PBM. The grinding media fill level was 80% of the grinding chamber volume. The mill was operated in circular mode. The feed container held a total of 1L, 600g of suspension were ground, which corresponded to about 500ml. The entire system was cooled by a cryostat so that a maximum of 23°C was measured at the outlet of the grinding chamber.

The specific energy demand in order to create nanoparticles by wet bead milling in stirred media mills was minimum 10,000 kJ/kg. The stress intensity (according to Kwade et al., 1996) was from $0.004 \cdot 10^{-3}$ Nm to $1 \cdot 10^{-3}$ Nm.

## Example 1

### Screening experiments of nanosuspensions

Methyl {4,6-diamino-2-[5-fluoro-1-(2-fluorobenzyl)-1H-pyrazolo[3,4-b]pyridin-3-yl]pyrimidin-5-yl}carbamate (Vericiguat, compound I) in crystalline form of modification I was milled in a wet bead milling process according to Examples 1-3 to a particle size within the nano scale (d50 after milling < 200 nm).

Fig. 1 shows the results of the screening experiments after applying a wet bead milling process in the planetary mill. The compound of formula (I) in crystalline form of modification I was produced according to example 13, method E of WO 2013/076168. Before nanomilling, the compound of formula (I) in crystalline form of modification I was micronized. For the screening experiments many different formulations were tested. For these experiments the duration of milling was 60 min. The material of the milling beads used was $ZrO_2$ with a size of the milling beads of 0.5 mm (0.4-0.6 mm).

In a first step, a broad screening of stabilizers revealed that not all excipients described in literature are suitable for producing nanosuspensions of this compound. It was found that sodium dodecyl sulfate (SDS) and Vitamin E TPGS as single stabilizers as well as the combination of two stabilizers like a surfactant and a polymer (e.g. SDS + polyvinylpyrrolidone K17 (PVP K17)) showed the best results. Eudragit® E PO, Egg lecithin, polyvinylacetate (PVA), sodium oleate and human serum albumin (HSA) were not suitable for nanomilling for various reasons. Human serum albumin, egg lecithin, hydroxypropylmethylcellulose acetate succinate (HPMC-AS) or sodium oleate resulted in unfavourable products with either too large particle or agglomeration phenomena.

The ratio of compound (I):one or more stabilizer was 16:1 to 1:2 w/w or 8:1 to 2:1 w/w. In the case that a combination of two stabilizers like a surfactant and a polymer (e.g. SDS plus polyvinylpyrrolidone or SDS plus ethylene oxide-propylene oxide block copolymer) is used, the ratio of compound (I):polymer was 16:1 to 1:2 w/w or 8:1 to 2:1 w/w and the concentration of surfactant was 0.1-0.2% w/v.

In the meantime, a new API quality of the compound of formula (I) in crystalline form of modification I was introduced, that was synthesized according to WO2020126983, published after the first filing date of the present application, and micronized. Thus, the screening was repeated with this new API quality and results could be reproduced (Fig. 1b).

Because SDS was also used for the further granulation process it was the preferred stabilizer for further investigation. However, also PVP VA 64, d-alpha tocopheryl polyethylene glycol 1000 succinate (Vitamin E TPGS), Cremophor® EL, PVP K30, and hydroxypropylcellulose (HPC) were used for further investigations in the Picoliq mill.

## Example 2

### Stability tests of nanosuspensions

#### a) Particle size directly after milling and over at least 7 days

Goal of these experiments was to evaluate the stability of the nanosuspensions over at least 7 days. Further on it was evaluated whether milling induced decomposition of the particles. For the nanomilling, the following parameters were applied: micronized compound (I):stabilizer = 2:1, content of compound (I) in the suspension 2%, milling bead material yttrium stabilized $ZrO_2$, milling bead size 0.4-0.6 mm, duration of milling 60 min, Picoliq mill.

**Table 1:** Results of particle size measurement (as d95) by dynamic light scattering (DLS) measurement of nanomilled Vericiguat (Picoliq mill) directly after milling and after storage of 7 days under ambient conditions

| | | Particle size as d95 [nm] | |
|---|---|---|---|
| **Sample** | **Excipients** | **After milling** | **7d@22°C** |
| 1 | SDS | 70 | 71 |
| 2 | PVP VA 64 | 280 | 197 |
| 3 | Vitamin E TPGS | 201 | 60 |
| 4 | Cremophor | 107 | 206 |
| 5 | PVP K30 | 84 | 195 |
| 6 | HPC | 453 | 699 |

The results show some small differences in the particle size although most of the dispersions are stable.

However, the HPC formulation shows a significant increase in d95 after 7 days at ambient temperature.

**b) Long-term measurement of particle growth over 13 weeks**

The stability of the different nanoparticles obtained in the screening of Example 1 was measured for up to 13 weeks at elevated temperature. The milling conditions were as follows: ratio of compound (I): stabilizer = 8:1; volumetric filling with milling beads 60%; milling bead material $ZrO_2$, milling bead size 0.4-0.6 mm; duration of milling 60 min; mill: planetary mill, storage at 40°C and 75% rh. The particle size distribution of the nanosuspension was measured via dynamic light scattering (DLS). DLS was performed with Zetasizer Nano-ZS (from Malvern Analytical) at suitable concentration (e.g. 0.2 mg/mL, dilution with demineralized water. As response, Dn(50) and polydispersity index (PDI) were obtained. The results are shown in Figure 2.

**Example 3**

**Scale up of the preparation of nanosuspensions**

The next step of the development of nanosuspensions was the scale up. For this, at least two problems had to be solved:
In a first step, the concentration of the compound of formula (I) (Vericiguat) in the nanosuspension had to be increased from 2% to 20%. This was mainly needed to reduce the drying effort and process time. A 2% suspension is too diluted to be preferred for a fluidized bed granulation. These experiments were carried out in a planetary mill.

In Figure 3 the results of these experiments in the planetary mill are shown. "LD particle size" indicates measurement of particle size by static light scattering (SLS). During the experiments it became clear that it was not possible to have a concentration of SDS higher than 5% due to gelling. Therefore, for a higher concentration of the compound of formula (I) in the nanosuspension, the ratio of the compound of formula (I):stabilizer had to be changed in favor of higher API share. The particle size measurement shows that a higher concentration of the compound of formula (I) in the nanosuspension of up to 20 % is possible without a significant increase in the particle size. On the contrary, the d90 value even slightly decreased with increasing concentrations of the compound of formula (I) and increasing ratios of compound (I): stabilizer.

The experiments showed that an increase of concentration of compound (I) was possible. However, it should be considered to start with a lower concentration of stabilizer. (see above, Example 3)

In a second step, the process had to be transferred to a larger mill to be able to produce a larger amount of product. For this purpose, the process was transferred to the Netzsch Labstar mill with a size of 120 ml. 60 g of nanosuspension were produced for further processing in the fluidized bed granulation (FBG). This experiment was done using the Netzsch mill in batch mode. The results of this experiment are shown in Figure 4.

The median particle size was 120 nm. The suspension was further processed by fluidized bed granulation.

**Example 4**

**Compound (I) in crystalline form of modification I after wet bead milling in Picoliq mill**

In order to analyze whether amorphization of the compound (I), employed in micronized, crystalline form of modification I in the wet bead milling process for producing nanosuspensions, occurs during wet bead milling, three nanosuspensions were produced by wet bead milling in the Picoliq mill under varying conditions, as shown in Table 2:

**Table 2:**

| | Suspension 1 | Suspension 2 | Suspension 3 |
|---|---|---|---|
| Composition compound (I) + SDS | 5:1 | 2:1 | 8:1 |
| Size of particles (D(n50) [nm] after milling | 136 | 189 | 108 |
| Duration of milling [min] | 35 | 10 | 60 |
| Yield [mg] | 44.77 | 36.21 | 99.44 |
| Rotational speed [rpm] | 5500 | 4000 | 7000 |
| Net weight of milling beads [g] | 46.2 | 56.8 | 35.5 |
| Percentage of compound (I) | 2 | 2 | 2 |

(continued)

| | Suspension 1 | Suspension 2 | Suspension 3 |
|---|---|---|---|
| Stabilizer | SDS | SDS | SDS |
| Percentage of stabilizer [m/v] | 0.4 | 1 | 0.25 |
| Temperature [°C] | 20.1-22.5 | 16.5-25.3 | 18.7-27.3 |

XRPD analysis of these three nanosuspensions is shown in Figures 5a to 5c. The XRPD analysis revealed that the crystalline from of modification I was preserved during wet bead milling even under high mechanical stress, such as long milling duration and high rotational speed, as exemplified for Suspension 3.

## Example 5

### Further Processing of Nanosuspensions to Granules and Tablets

A wet granulation technique (fluidized bed granulation) was used to improve of the employed pre-blend. The active compound is suspended in the granulation liquid and sprayed on the pre-blend to uniformly distribute the active compound in the resulting granulate.

### Process steps up to the final tablet formulation

To investigate the influence of particle size on the bioavailability of methyl {4,6-diamino-2-[5-fluoro-1-(2-fluorobenzyl)-1H-pyrazolo[3,4-b]pyridin-3-yl]pyrimidin-5-yl}carbamate (Vericiguat, compound (I)), several formulations were manufactured using suspensions with drug substance that was first micronized and then nanomilled and suspensions with drug substance that was only micronized. The compositions of these suspensions are outlined in the next paragraph. In the context of the present invention micronization is carried out for example by comminution in a spiral jet mill or a fluidized-bed opposed jet mill.

First step - preparation of suspension with micronized drug substance: For incorporating the micronized drug substance in a suspension, the binder hydroxypropylmethylcellulose (HPMC 5cP) and the wetting agent sodium dodecyl sulfate (SDS) were dissolved in water. After a clear solution was obtained, the micronized methyl {4,6-diamino-2-[5-fluoro-1-(2-fluorobenzyl)-1H-pyrazolo[3,4-b]pyridin-3-yl]pyrimidin-5-yl}carbamate (Vericiguat, compound (I)) was suspended in this binder solution and distributed homogeneously (compare **Table 3**, "suspension").

Second step - preparation of granules using the suspension with micronized drug substance: In order to generate granules, the resulting suspension was sprayed onto a pre-mix of the fillers cellulose microcrystalline (Avicel PH 101) and lactose monohydrate and one part of the disintegrant croscarmellose sodium (AcDiSol) using the fluidized bed granulation technique. The granules were dried and sieved (mesh size 0.8 mm) (compare **Table 3**, "granules").

Third step - preparation of tablets on the basis of the granules containing micronized drug substance: The resulting granules were post-blended with the second part of the disintegrant croscarmellose sodium and the lubricant magnesium stearate in two steps. A part of this ready-to-press blend was compressed to tablets (round format with a diameter of 9 mm) or to mini-tablets (with a diameter of 1.2 mm) (compare **Table 3,** "tablet").

Using this 3 step-procedure, two different batches of tablets were manufactured differing in dose (compare **Table 3,** batches 2 and 4).

A comparable approach was implemented for the use of micronized plus nanomilled drug substance.

First step - preparation of suspension with micronized plus nanomilled drug substance: For incorporating micronized plus nanomilled compound (I), physically stabilized with sodium dodecyl sulfate (SDS), into granules, an aqueous suspension of micronized plus nanomilled compound (I) and sodium dodecyl sulfate (SDS) was taken and mixed with an aqueous solution of hydroxypropylmethylcellulose (HPMC 5cP) in water in the relative amounts given in Table 3 below. Of the amount of water used, 30% is used in the preparation of nanoparticles and 70% is used in the preparation of the aqueous solution of binder (HPMC 5cP) (compare **Table 3,** "suspension").

Second step - preparation of granules using the suspension of micronized plus nanomilled drug substance: The resulting suspension was sprayed onto a premix of the fillers cellulose microcrystalline and lactose monohydrate and one part of the disintegrant croscarmellose sodium (AcDiSol) using the fluidized bed granulation technique. The granules (pre-blend) were dried and sieved (mesh size 0.8 mm) (compare Table 3, "granules"). The granules contain nanoparticles in dried form. The dispersing agent (here: water) was removed in the drying step.

Third step - preparation of tablets on the basis of the granules containing micronized plus nanomilled drug substance: The resulting granules were post-blended with the second part of the disintegrant croscarmellose sodium (AcDiSol) and the lubricant magnesium stearate in two steps. A part of this ready-to-press blend was compressed to

tablets (round format with a diameter of 9 mm). For the administration of tablets in *in vivo* studies in rats (Example 6b), mini-tablets were compressed (diameter of 1.2 mm) (compare **Table 3,** "tablet"). The tablets contain nanoparticles in dried form that do not contain dispersing agent (here: water) anymore.

Using this 3 step-procedure, two different batches of tablets using micronized and nanomilled compound (I) were manufactured differing only in the dose strength (compare **Table 3,** batches 1 and 3).

**Table 3:** Composition of tablets containing compound (I) (Vericiguat) using micronized and nanomilled drug substance (mg/tablet, diameter 9 mm)

| | batch 1 | batch 2 | batch 3 | batch 4 | | | |
|---|---|---|---|---|---|---|---|
| **Cpd. (I) micron.** | --- | 10.0 | --- | 15.0 | | | |
| **Cpd. (I) nanomilled,** | 10.0 | --- | 15.0 | --- | | | |
| **stabilized with SDS** | 2.5 | | 3.75 | | | | |
| **HPMC 5cP** | 8.4 | 8.4 | 8.5 | 8.5 | Suspension | | |
| **SDS** | --- | 2.5 | --- | 3.75 | | | |
| **Σ of Water (process aid)** | 130.0 | 130.0 | 190.0 | 190.0 | | | |
| **Avicel PH 101** | 84.0 | 84.0 | 80.0 | 80.0 | | Granules | |
| **Lactose monohydrate** | 115.7 | 115.7 | 110.25 | 110.25 | | | |
| **AcDiSol** | 8.5 | 8.5 | 10.0 | 10.0 | | | |
| **AcDiSol** | 8.5 | 8.5 | 10.0 | 10.0 | | | Tablet |
| **Magnesium stearate** | 2.4 | 2.4 | 2.5 | 2.5 | | | |
| **SUM** | **240.0** | **240.0** | **240.0** | **240.0** | | | |
| **Hardness [N]** | 91 | 94 | 87 | 97 | | | |
| **Disintegration time max [sec]** | 181 | 143 | 141 | 293 | | | |

The manufactured granules of the batches 3 and 4 (15 mg dose strength) were characterised via flow through experiments (n = 2 ± SD (1 mg API per cell, 2 ml/min flow rate)) in a mini flow through cell dissolution apparatus (USP Convention 2011, Revision Bulletin, Official Feb 1, 2012, General Chapter <711> Dissolution) in three different media (phosphate buffer pH 6.8, FaSSIF and FeSSIF). The results are summarized in Figures 6 and 7.

Figures 6 and 7 clearly demonstrate the increased drug release of granules made with micronized plus nanomilled compound (I) (Vericiguat) in comparison to granules produced with compound (I) (Vericiguat), that was only micronized (composition according to **Table 3**) which is the only difference in the investigated batches. This superiority of the micronized plus nanomilled compound (I) (Vericiguat) is independent of the media tested.

Additionally, the tablets of all 4 manufactured batches (compare **Table 3**) were characterised via drug dissolution behaviour using the USP II paddle apparatus (USP Convention 2011, Revision Bulletin, Official Feb 1, 2012, 711 Dissolution) with 900mL 0.01N HCl, mixing with 75 rpm. A comparison is given in Figure 8 for the 10 mg dose and in Figure 9 for the 15 mg dose.

The percentage of dissolved drug is higher in the two tablet batches with the micronized plus nanomilled compound (I) (Vericiguat) in comparison to both batches with micronized drug substance, independent of the dose strength investigated.

Further, the 15 mg tablets containing either micronized plus nanomilled or only micronized compound (I) (Vericiguat), with the composition according to Table 4, were characterised via drug dissolution behaviour using the USP II paddle apparatus (USP Convention 2011, Revision Bulletin, Official Feb 1, 2012, 711 Dissolution) with 500mL FeSSIF, mixing with 75 rpm. The dissolution data are given in Figure 11. The data clearly demonstrate the increased drug release using the micronized plus nanomilled compound (I) (Vericiguat) in comparison to the micronized compound (I) (Vericiguat) which is the only difference in the investigated batches.

**Table 4:** Composition of tablets containing compound (I) (Vericiguat) using micronized and micronized plus nanomilled drug substance (mg/tablet, diameter 9 mm)

| | batch 5 | batch 6 | | | |
|---|---|---|---|---|---|
| **Cpd. (I) micron.** | --- | 15.0 | Suspension | | |
| **Cpd. (I) nanomilled, stabilized** | 15.0 | --- | | | |
| **with Polysorbate 20** | 5.0 | | | | |
| **HPMC 3cP** | 8.5 | 8.5 | | | |
| **Polysorbate 20** | --- | 5.0 | | | |
| **Σ of Water (process aid)** | 188.0 | 188.0 | | | |
| **Avicel PH 101** | 80.0 | 80.0 | | Granules | |
| **Lactose monohydrate** | 99.0 | 99.0 | | | |
| **AcDiSol** | 10.0 | 10.0 | | | |
| **AcDiSol** | 20.0 | 20.0 | | | Tablet |
| **Magnesium stearate** | 2.5 | 2.5 | | | |
| **SUM** | **240.0** | **240.0** | | | |
| **Hardness [N]** | 94 | 95 | | | |
| **Disintegration time max [sec]** | 284 | 178 | | | |

## Example 6

### In vivo experiments

### a) Nanosuspensions

All *in vivo* studies were performed in catheterized male Wistar rats (n = 3) in line with the German Protection of Animals Act. Three doses were applied in different formulations (suspensions, granules, mini-tablets): 0.3, 1.0 and 3.0 mg/kg (for granules: 2.1 mg/kg as highest dose), bracketing the therapeutic dose range. The application volume for solutions and suspensions was 5 mL/kg. Plasma samples were collected over a period of 48 hours post-dose and analyzed by LC-MS/MS. Pharmacokinetic parameters were estimated from plasma concentration-time profiles with standard PK software. The relative bioavailability compares two different formulations applied via the same administration route at a normalized dose and was calculated as the AUC ratio obtained from the different study arms.

As comparison, the test compound (compound (I)) was administered orally as a solution containing compound (I) which is unlikely to precipitate *in vivo* (100% PEG or PEG/EtOH/water (40/10/50, V/V/V)). As a surrogate for a disintegrated standard tablet, a suspension containing a 0.5% tylose solution was administered, in which the compound is suspended as micronized material. The below two nanosuspensions were tested in different dose strengths:

a) SDS-stabilized nanosuspension

- a. 0.6 mg/mL micronized plus nanomilled compound (I) + 0.3 mg/mL SDS solution
- b. 1 mg/mL micronized plus nanomilled compound (I) + 0.5 mg/mL SDS solution
- c. 6 mg/mL micronized plus nanomilled compound (I) + 3 mg/mL SDS solution

b) Vitamin E TPGS-stabilized nanosuspension

- a. 0.6 mg/mL micronized plus nanomilled compound (I) + 0.3 mg/mL Vitamin E TPGS solution
- b. 1 mg/mL micronized plus nanomilled compound (I) + 0.5 mg/mL Vitamin E TPGS solution
- c. 6 mg/mL micronized plus nanomilled compound (I) + 3 mg/mL Vitamin E TPGS solution

Subsequently to the animal study, the particle sizes of the applied nanosuspensions were checked to verify that no critical change of particle size had occurred. The bioavailability of the nanosuspensions is shown in Table 5.

Table 5: Bioavailability of Nanosuspensions

| | AUC norm | | | $C_{max}$ norm | | |
|---|---|---|---|---|---|---|
| | kg*h/L | | | kg/L | | |
| | 0.3 | 1 | 3 | 0.3 | 1 | 3 |
| **Solution (100% PEG)** | 3.45 | | 4.61 | 0.376 | | 0.349 |
| **Solution (PEG/EtOH/Water)** | | 3.19 | | | 0.343 | |
| **Tylose suspension (microcrystalline material)** | 2.12 | | 1.18 | 0.267 | | 0.126 |
| **Nanosuspension Vitamin E TPGS** | 2.55 | 2.01 | 2.13 | 0.25 | 0.263 | 0.222 |
| **Nanosuspension SDS** | 2.34 | 2.3 | 2.78 | 0.299 | 1.3 | 0.31 |

**b) Granules and tablets**

Granules based on microsuspensions or nanosuspensions (manufactured by fluidized bed granulation using compound (I) in micronized or micronized plus nanomilled form) were administered in small capsules (dose: 0.3 and 2.1 mg/kg due to limited filling volume, composition see Table 3). Mini-tablets (diameter = 1.2 mm), made from above granules, were also administered in small capsules. The dose of 0.3 mg/kg corresponded to the ratios of ingredients of 1 tablet of the 15 mg granules, whereas consequently the dose of 3 mg/kg corresponded to 10 tablets.

Above described granules based on the nanosuspension of the compound of formula (I) were compared to granules based on a microsuspension of the compound of formula (I) and to the compound of formula (I) as PEG solution *in vivo.* Dose-proportionality was observed. The results are shown in Tables 5 and 6 and in Fig. 10.

Table 6: Bioavailability of Granules

| | AUC norm [kg*h/L] | | $C_{max}$ norm [kg/L] | |
|---|---|---|---|---|
| **Dose** | 0.3 mg/kg | 2.1 mg/kg | 0.3 mg/kg | 2.1 mg/kg |
| **Granule micro batch** | 1.26 | 1.42 | 0.168 | 0.158 |
| **Granule nano batch** | 2.14 | 1.9 | 0.135 | 0.185 |

Table 7: Bioavailability of Tablets

| | AUC norm [kg*h/L] | | $C_{max}$ norm [kg/L] | |
|---|---|---|---|---|
| **Dose** | 0.3 mg/kg | 3 mg/kg | 0.3 mg/kg | 3 mg/kg |
| **Micro Tablet** | 3.18 | 1.53 | 0.309 | 0.192 |
| **Nano Tablet** | 3.39 | 2.36 | 0.245 | 0.318 |

In lower dose (0.3 mg/kg), micro- and nano-formulations were comparable. In higher dose (2.1 or 3 mg/kg), the nano-formulation was superior, as shown by an increase in AUC and $C_{max}$. Superiority of the formulations containing micronized plus nanomilled compound (I) over formulations containing compound (I), which was only micronized, was shown for every formulation type, i.e. suspension, granules, and tablets.

## Example 7

## Screening experiments on stability of nanosuspensions and reconstitution after drying

In order to analyze, whether nano-size particles are still present after reconstitution of dried material that was obtained by drying of nanosuspensions, the following screening experiments were performed. Different active ingredient/stabilizer combinations were nanomilled in a planetary ball mill (PBM) of type Pulverisette 5, as described in Method A, with the deviation that 5 ml vials were used, and the grinding time was 60 minutes at 400 rotations per minute.

The particle size distribution was measured directly after milling. For measurement of suspension stability, the nanosuspensions were stored for 7 days at 40°C at room conditions without stress (no agitation).

For measurement of stability of nanoparticles after drying and reconstitution, the nanosuspensions were dried

directly after milling in a drying chamber at 40°C for 14-18 hours. Water was added onto the dried film and it was mixed with a magnetic stirrer for max. 5min. The particle size distribution was measured. These tests were performed with different combinations of HPC or PVP K30 and SDS as stabilizer and lactose as matrix former in the drying process. Results are shown in Tables 7 and 8.

**Table 7: 5% Vericiguat with PVP K30 / SDS / Lactose**

| Excipients | | | Drug load in solid | Analysis directly after milling | Analysis after 7days storage | Analysis after drying / redispersion |
|---|---|---|---|---|---|---|
| **PVP K30** | SDS | Lactose | | PSD (laser diffraction) | Difference in particle size (d10, d50, d90) | Difference in particle size (d10, d50, d90) |
| 1.0% | 0.1% | 0% | 100% | d50 < 200nm | < +20% | d90 > 600μm |
| 1.0% | 0.1% | 1% | 83% | d50 < 200nm | < +20% | d90 > 90μm |
| 1.0% | 0.1% | 5% | 50% | d50 < 200nm | < +20% | d90 > 80μm |
| 1.0% | 0.1% | 15% | 25% | d50 < 200nm | < +20% | d90 > 100μm |
| 2.5% | 0.1% | 0% | 100% | d50 < 200nm | < +20% | d90 > 50μm |
| 2.5% | 0.1% | 1% | 83% | d50 < 200nm | < +20% | d90 >10μm |
| 2.5% | 0.1% | 5% | 50% | d50 < 200nm | < +20% | d90 > 3μm |
| 2.5% | 0.1% | 15% | 25% | d50 < 200nm | < +20% | d90 > 40μm |
| 5.0% | 0.1% | 0% | 100% | d50 < 200nm | < +20% | d90: 113% |
| 5.0% | 0.1% | 1% | 83% | d50 < 200nm | < +20% | < +20% |
| 5.0% | 0.1% | 5% | 50% | d50 < 200nm | < +20% | d90: 21% |
| 5.0% | 0.1% | 15% | 25% | d50 < 200nm | < +20% | d90: 25% |

**Table 8: 5% Vericiguat with HPC / SDS / Lactose**

| Excipients | | | Drug load in solid | Analysis directly after milling | Analysis after 7days storage | Analysis after drying / redispersion |
|---|---|---|---|---|---|---|
| **HPC** | SDS | Lactose | | PSD (laser diffraction) | Difference in particle size (d10, d50, d90) | Difference in particle size (d10, d50, d90) |
| 1.0% | 0.1% | 0% | 100% | d50 < 200nm | < +20% | d90 > 25μm |
| 1.0% | 0.1% | 1% | 83% | d50 < 200nm | < +20% | d90 > 14μm |
| 1.0% | 0.1% | 5% | 50% | d50 < 200nm | < +20% | d90 > 4μm |
| 1.0% | 0.1% | 15% | 25% | d50 < 200nm | < +20% | d90 > 21μm |
| 2.5% | 0.1% | 0% | 100% | d50 < 200nm | < +20% | d90: 500% |
| 2.5% | 0.1% | 1% | 83% | d50 < 200nm | < +20% | d90: 25% |
| 2.5% | 0.1% | 5% | 50% | d50 < 200nm | < +20% | d90: 24% |
| 2.5% | 0.1% | 15% | 25% | d50 < 200nm | < +20% | < +20% |
| 5.0% | 0.1% | 0% | 100% | d50 < 200nm | < +20% | d90: 28% |
| 5.0% | 0.1% | 1% | 83% | d50 < 200nm | < +20% | d90: 23% |
| 5.0% | 0.1% | 5% | 50% | d50 < 200nm | < +20% | < +20% |
| 5.0% | 0.1% | 15% | 25% | d50 < 200nm | < +20% | d90: 28% |

## Figures

Fig. 1a shows the results of the screening experiments of nanosuspensions made with the compound of formula (I) after wet bead milling in the planetary mill with different stabilizers (Example 1.

Fig. 1b shows the results of the screening experiments of nanosuspensions made with the compound of formula (I), synthesized according to WO2020126983, published after the first filing date of the present application, after wet bead milling in the planetary mill with different stabilizers (Example 1).

Fig. 2 shows the long-term stability of the different nanoparticles obtained in the screening of Example 1, measured for up to 13 weeks at elevated temperature. The milling conditions were as follows: ratio of compound (I):stabilizer = 5:1; volumetric filling with milling beads 50%; milling bead size 0.3-0.4 mm; duration of milling 90 min; mill: planetary mill, storage at 40°C. Particle size is given as Dn(50) and polydispersity index (PDI).

Fig. 3 shows the particle size distribution for different concentrations of micronized and nanomilled compound (I) and compound (I):stabilizer ratios milled in the planetary mill. API = compound (I) (Example 3, scale up).

Fig. 4 shows the particle size distribution after nanomilling of compound (I) (Vericiguat) (20% API suspended in relation to total mass). The data are given as volume fraction Q3 (percentage by volume, vol%, Q3 indicates that the particles were measured three dimensional, X% indicates the percentage of particles that are smaller than the indicated size). The Vericiguat material of both curves was micronized and not sonicated. Left curve: nanomilled in the Netzsch mill with 3.3% SDS for 90 min. Right curve: no nanomilling.

Fig. 5a shows the XRPD measured for Suspension 1 of Example 4. The lower diagram shows the XRPD of the compound (I) in crystalline modification I as reference, the upper diagram shows the XRPD of the compound (I) after wet bead milling under the conditions shown for Suspension 1 in Table 2. The comparison shows that no amorphization occurs during the wet bead milling procedure.

Fig. 5b shows the XRPD measured for Suspension 2 of Example 4. The lower diagram shows the XRPD of the compound (I) in crystalline modification I as reference, the upper diagram shows the XRPD of the compound (I) after wet bead milling under the conditions shown for Suspension 2 in Table 2. The comparison shows that no amorphization occurs during the wet bead milling procedure.

Fig. 5c shows the XRPD measured for Suspension 3 of Example 4. The lower diagram shows the XRPD of the compound (I) in crystalline modification I as reference, the upper diagram shows the XRPD of the compound (I) after wet bead milling under the conditions shown for Suspension 3 in Table 2. The comparison shows that no amorphization occurs during the wet bead milling procedure.

Fig. 6 shows the amounts of compound (I) (Vericiguat), as granules, released in the flow through cell using three different media. Fig. 7 shows the cumulative amounts of compound (I) (Vericiguat), as granules, released measured with the flow through cell using three different media. Figures 6 and 7 both clearly demonstrate the increased drug release using the nanomilled compound (I) (Vericiguat) in comparison to the micronized compound (I) (Vericiguat) which is the only difference in the investigated batches. This superiority of the nanomilled compound (I) (Vericiguat) is independent of the media tested.

Fig. 8 shows the drug dissolution velocity data of tablets containing compound (I) (Vericiguat) in the dose strengths 10 mg where micronized or nanomilled drug substance was incorporated, respectively. The drug dissolution velocity was measured in 0.01N HCl (mean of n=6).

Fig. 9 shows the drug dissolution velocity data of tablets containing compound (I) (Vericiguat) in the dose strengths 15 mg where micronized or nanomilled drug substance was incorporated, respectively. The drug dissolution velocity was measured in 0.01N HCl (mean of n=6).

Fig. 10 shows an overview about all *in vivo* results obtained from different formulations containing compound (I) (solution, suspension, granules, mini-tablets). Low dose: 0.3 mg/kg, mid dose: 1 mg/kg (only solution), high dose: 2.1 mg/kg for granules, 3 mg/kg for solution, suspension (two different particle sizes utilized: tylose suspension with microcrystalline material and suspension with nano-sized compound (I)) and mini-tablets (manufactured out of either micronized or micronized plus nanomilled compound (I)).

Fig. 11 shows dissolution data of a tablet core (without coating) containing 15mg of compound (I) either in micronized or micronized plus nanomilled form. The dissolution profiles were measured in a biorelevant medium (FeSSIF) where non-sink conditions were present. "Non-sink conditions" means that less than the three-fold amount of the dose contained in the tablet is solved in the release medium. This leads to a higher discrimination. Looking at the profiles, the formulation with nanomilled compound (I) is clearly superior to the one with micronized compound (I), the particle size of compound (I) being the only difference in both tablet cores.

Fig 12a and Fig 12b show confocal laser scanning microscopy (CLSM) pictures of granules containing micronized (Fig

12a, #WU-000704-01) and nanomilled (Fig. 12b, #WU-000704-02) compound of the formula (I), respectively. Fluorescence was induced at 405nm, emission was measured at 420 - 500nm, gain 550, objective HC PL APO CS2 63x/1.40 OIL. Fig. 12a clearly shows light spots representing fluorescent compound of the formula (I) that was only micronized. In Fig. 12b, representing fluorescent compound of the formula (I) clearly shows a much smoother surface with no light spots.

Citations

Butler JM, Dressman JB. The developability classification system: application of biopharmaceutics concepts to formulation. J Pharm Sci (2010) 99:4940-4954.

Choi YH, Han H-K. Review. Nanomedicines: current status and future perspectives in aspect of drug delivery and pharmacokinetics. Journal of Pharmaceutical Investigation (2018) 48:43-60.

Desai PP, Date AA, Patravale VB. Overcoming poor oral bioavailability using nanoparticle formulations - opportunities and limitations. Drug Discovery Today, Technologies (2012) 9:e87-e95.

Follmann M et al.. Discovery of the Soluble Guanylate Cyclase Stimulator Vericiguat (BAY 1021189) for the Treatment of Chronic Heart Failure. J Med Chem (2017) 60:5146-5161.

George M, Gosh I. Identifying the correlation between drug/stabilizer properties and critical quality attributes (CQAs) of nanosuspension formulation prepared by wet milling technology. Eur J Phar Sci (2013) 48:142-152.

Hunter JR, Preedy VR. Nanocrystal Formulations for Improved Delivery of Poorly Soluble Drugs. Nanomedicine in Health and Disease. CRC press (2011) 1st edition, chapter 5.

Jermain SV, Brough, C, Williams, RO. Amorphous solid dispersions and nanocrystal technologies for poorly water-soluble drug delivery-An update. International Journal of Pharmaceutics (2018) 535:379-392.

Kumar MP, Rao YM, Apte S. Formulation of Nanosuspensions of Albendazole for Oral Administration. Current Nanoscience (2008) 4:53-58.

Kwade A, Blecher L, Schwedes J. Motion and stress intensity of grinding beads in a stirred media mill. Part 2: Stress intensity and its effect on comminution. Powder technology (1996) 86:69-76.

Li M, Azad M, Davé R, Bilgili E. Review. Nanomilling of Drugs for Bioavailability Enhancement: A Holistic Formulation-Process Perspective. Pharmaceutics (2016) 8:17.

Merisko-Liversidge, EM, Liversidge, GG. Drug nanoparticles: formulating poorly water-soluble compounds Toxicol. Pathol. (2008) 36, 43-48.

Van Eerdenbrugh B, Van den Mooter G, Augustijns P. Top-down production of drug nanocrystals: Nanosuspension stabilization, miniaturization and transformation into solid products. Int J Pharm (2008) 364:64-75.

## Claims

1. Stable nanosuspension comprising nanoparticles of methyl {4,6-diamino-2-[5-fluoro-1-(2-fluorobenzyl)-1H-pyrazolo [3,4-b]pyridin-3-yl]pyrimidin-5-yl}carbamate of the formula (I)

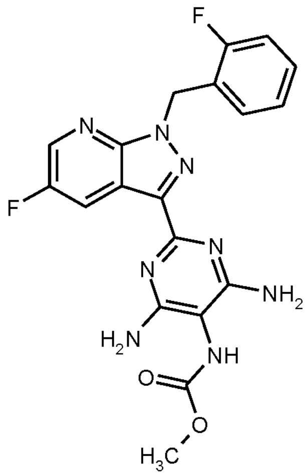

(I)

in crystalline form of modification I, **characterized in that** the x-ray diffractogram of the compound exhibits peak maxima of the 2 theta angle at 5.9, 6.9, 22.7, and one or more stabilizer(s) in a dispersing agent,
wherein the one or more stabilizer is polyvinylpyrrolidone (PVP) in combination with sodium dodecylsulfate (SDS), or selected from the group consisting of vinylpyrrolidone-vinyl acetate copolymer, ethylene oxide-propylene oxide block copolymer, sodium dodecylsulfate (SDS), hydroxypropylmethylcellulose (HPMC), polysorbate, hydroxypropylcellulose (HPC), polyoxyl-35 castor oil, polyoxyl 15 hydroxystearate, Na-desoxycholate, and combinations thereof,
the maximum concentration of the one or more stabilizer(s) is the solubility limit of the stabilizer(s) in the suspension,
the dispersing agent is selected from the group consisting of water, primary, secondary, and tertiary alcohols, and polyvalent alcohols,
the nanoparticles have an average particle size, expressed as d50, of 500 nm or less, and
the average particle size, expressed as d50, remains at 500 nm or less at storage for at least one week at a temperature of at least 40°C.

**2.** Stable nanosuspension according claim 1, wherein the one or more stabilizer is selected from the group consisting of sodium dodecylsulfate, polyvinylpyrrolidone K10 to K50 in combination with sodium dodecylsulfate (SDS), hydroxypropylmethylcellulose, polysorbate 20-80, and combinations thereof.

**3.** Stable nanosuspension according to claim 1 or 2, wherein the ratio of the compound of formula (I):one or more stabilizer(s) is 16:1 to 1:2 w/w.

**4.** Stable nanosuspension according to any of claims 1 to 3, wherein the nanoparticles have an average particle size, expressed as d50, of 300 nm or less.

**5.** Stable nanosuspension according to any of claims 1 to 4, wherein the average particle size, expressed as d50, remains at 300 nm or less at storage for at least one week at a temperature of at least 40°C.

**6.** Process for preparing the stable nanosuspension according to any of claims 1 to 5, comprising the steps of

a. suspending the compound of formula (I) in crystalline form of modification I in a dispersing agent selected from the group consisting of water, primary, secondary, and tertiary alcohols, and polyvalent alcohols, and one or more stabilizer according to claim 1 or 2, wherein the maximum concentration of the one or more stabilizer(s) is the solubility limit of the stabilizer(s) in the suspension;
b. wet bead milling the suspension generated in step a. with a specific energy input of 10,000 kJ/kg or more and a stress intensity of $0.004 \cdot 10^{-3}$ Nm to $1 \cdot 10^{-3}$ Nm, wherein the milling beads used have a size of 0.05-0.8 mm.

**7.** Process according to claim 6, wherein in step a., the compound of formula (I) in crystalline form of modification I, is micronized in a first step before suspending it in a dispersing agent and one or more stabilizer.

**8.** Process according to claim 6, wherein in step a., the compound of formula (I) in crystalline form of modification I, is

milled in a first wet bead milling step in the presence of milling beads having a size of 1-2 mm before suspending it in a dispersing agent and one or more stabilizer.

**9.** Process according to any of claims 6 to 8, wherein the milling beads are made from a material selected from the group selected from ceramics, glass, polymers, and steel.

**10.** Process according to any of claims 6 to 9, wherein the volumetric filling with milling beads is 50-85% v/v.

**11.** Dried nanoparticles comprising methyl {4,6-diamino-2-[5-fluoro-1-(2-fluorobenzyl)-1H-pyrazolo[3,4-b]pyridin-3-yl] pyrimidin-5-yl}carbamate of the formula (I) in crystalline form of modification I, **characterized in that** the x-ray diffractogram of the compound exhibits peak maxima of the 2 theta angle at 5.9, 6.9, 22.7, and one or more stabilizer(s) in a ratio of the compound of formula (I):one or more stabilizer(s) of 16:1 to 1:2 w/w, wherein the dried nanoparticles have an average particle size expressed as d50 of 500 nm or less, the one or more stabilizer(s) are as defined in claim 1, and the dried nanoparticles are made by drying the stable nanosuspensions prepared by the process of claim 6.

**12.** Pharmaceutical composition in solid form comprising dried nanoparticles of methyl {4,6-diamino-2-[5-fluoro-1-(2-fluorobenzyl)-1H-pyrazolo[3,4-b]pyridin-3-yl]pyrimidin-5-yl}carbamate of the formula (I) in crystalline form of modification I, **characterized in that** the x-ray diffractogram of the compound exhibits peak maxima of the 2 theta angle at 5.9, 6.9, 22.7, and one or more stabilizer(s) having an average particle size, expressed as d50, of 500 nm or less, made with the nanoparticles of claim 11.

**13.** Pharmaceutical composition according to claim 12, wherein the solid form is selected from the group consisting of granules and tablets.

**14.** Dried nanoparticles or a pharmaceutical composition according to any of claims 11 to 13, wherein the dried nanoparticles or the pharmaceutical composition do not contain a dispersing agent.

**Patentansprüche**

**1.** Stabile Nanosuspension, umfassend Nanopartikel von Methyl-{4,6-diamino-2-[5-fluor-1-(2-fluorbenzyl)-1H-pyrazolo[3,4-b]pyridin-3-yl]pyrimidin-5-yl}carbamat der Formel (I)

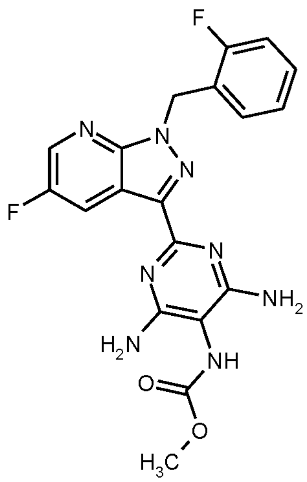

(I)

in kristalliner Form der Modifikation I, **dadurch gekennzeichnet, dass** das Röntgendiffraktogramm der Verbindung Peakmaxima des 2-Theta-Winkels bei 5,9, 6,9, 22,7 aufweist, und einen oder mehrere Stabilisatoren in einem Dispergiermittel,

wobei der eine bzw. die mehreren Stabilisatoren Polyvinylpyrrolidon (PVP) in Kombination mit Natriumdodecylsulfat (SDS) sind oder aus der Gruppe bestehend aus Vinylpyrrolidon-Vinylacetat-Copolymer, Ethylenoxid-Propylenoxid-Blockcopolymer,

Natriumdodecylsulfat (SDS), Hydroxypropylmethylcellulose (HPMC), Polysorbat, Hydroxypropylcellulose (HPC), Polyoxyl-35-rizinusöl, Polyoxyl-15-hydroxystearat, Na-Desoxycholat und Kombinationen davon ausgewählt sind, die maximale Konzentration des einen bzw. der mehreren Stabilisatoren die Löslichkeitsgrenze des bzw. der Stabilisatoren in der Suspension ist,
das Dispergiermittel aus der Gruppe bestehend aus Wasser, primären, sekundären und tertiären Alkoholen und mehrwertigen Alkoholen ausgewählt ist,
die Nanopartikel eine durchschnittliche Teilchengröße, ausgedrückt als d50, von 500 nm oder weniger aufweisen und
die durchschnittliche Teilchengröße, ausgedrückt als d50, bei Lagerung für mindestens eine Woche bei einer Temperatur von mindestens 40 °C bei 500 nm oder weniger bleibt.

**2.** Stabile Nanosuspension nach Anspruch 1, wobei der eine bzw. die mehreren Stabilisatoren aus der Gruppe bestehend aus Natriumdodecylsulfat, Polyvinylpyrrolidon K10 bis K50 in Kombination mit Natriumdodecylsulfat (SDS), Hydroxypropylmethylcellulose, Polysorbat 20-80 und Kombinationen davon ausgewählt sind.

**3.** Stabile Nanosuspension nach Anspruch 1 oder 2, wobei das Verhältnis der Verbindung der Formel (I) zu einem oder mehreren Stabilisatoren 16:1 bis 1:2 w/w beträgt.

**4.** Stabile Nanosuspension nach einem der Ansprüche 1 bis 3, wobei die Nanopartikel eine durchschnittliche Teilchengröße, ausgedrückt als d50, von 300 nm oder weniger aufweisen.

**5.** Stabile Nanosuspension nach einem der Ansprüche 1 bis 4, wobei die durchschnittliche Teilchengröße, ausgedrückt als d50, bei Lagerung für mindestens eine Woche bei einer Temperatur von mindestens 40 °C bei 300 nm oder weniger bleibt.

**6.** Verfahren zur Herstellung der stabilen Nanosuspension nach einem der Ansprüche 1 bis 5, das folgende Schritte umfasst:

a. Suspendieren der Verbindung der Formel (I) in kristalliner Form der Modifikation I in einem Dispergiermittel, das aus der Gruppe bestehend aus Wasser, primären, sekundären und tertiären Alkoholen und mehrwertigen Alkoholen ausgewählt wird, und einem oder mehreren Stabilisatoren nach Anspruch 1 oder 2, wobei die maximale Konzentration des einen bzw. der mehreren Stabilisatoren die Löslichkeitsgrenze des einen bzw. der mehreren Stabilisatoren in der Suspension ist;
b. Nassperlmahlen der in Schritt a. erzeugten Suspension mit einem spezifischen Energieeintrag von 10.000 kJ/kg oder mehr und einer Beanspruchungsintensität von 0,004 x $10^{-3}$ Nm bis 1 $\times$ $10^{-3}$ Nm, wobei die verwendeten Mahlperlen eine Größe von 0,05-0,8 mm aufweisen.

**7.** Verfahren nach Anspruch 6, wobei in Schritt a. die Verbindung der Formel (I) in kristalliner Form der Modifikation I in einem ersten Schritt mikronisiert wird, bevor sie in einem Dispergiermittel und einem oder mehreren Stabilisatoren suspendiert wird.

**8.** Verfahren nach Anspruch 6, wobei in Schritt a. die Verbindung der Formel (I) in kristalliner Form der Modifikation I in einem ersten Nassperlmahlschritt in Gegenwart von Mahlperlen mit einer Größe von 1-2 mm gemahlen wird, bevor sie in einem Dispergiermittel und einem oder mehreren Stabilisatoren suspendiert wird.

**9.** Verfahren nach einem der Ansprüche 6 bis 8, wobei die Mahlperlen aus einem aus der Gruppe bestehend aus Keramik, Glas, Polymeren und Stahl ausgewählten Material bestehen.

**10.** Verfahren nach einem der Ansprüche 6 bis 9, wobei die volumetrische Füllung mit Mahlperlen 50 - 85 % v/v beträgt.

**11.** Getrocknete Nanopartikel, umfassend Methyl-{4,6-diamino-2-[5-fluor-1-(2-fluorbenzyl)-1H-pyrazolo[3,4-b]pyridin-3-yl]pyrimidin-5-yl}carbamat der Formel (I) in kristalliner Form der Modifikation I, **dadurch gekennzeichnet, dass** das Röntgendiffraktogramm der Verbindung Peakmaxima des 2-Theta-Winkels bei 5,9, 6,9, 22,7 aufweist, und einen oder mehrere Stabilisatoren in einem Verhältnis der Verbindung der Formel (I) zu einem bzw. mehreren Stabilisatoren von 16:1 bis 1:2 w/w, wobei die getrockneten Nanopartikel eine durchschnittliche Teilchengröße, ausgedrückt als d50, von 500 nm oder weniger aufweisen, der eine bzw. die mehreren Stabilisatoren wie in Anspruch 1 definiert sind und die getrockneten Nanopartikel durch Trocknen der durch das Verfahren nach Anspruch 6 hergestellten stabilen Nanosuspensionen hergestellt werden.

**12.** Pharmazeutische Zusammensetzung in fester Form, umfassend getrocknete Nanopartikel von Methyl-{4,6-diamino-2-[5-fluor-1-(2-fluorbenzyl)-1H-pyrazolo[3,4-b]pyridin-3-yl]pyrimidin-5-yl}carbamat der Formel (I) in kristalliner Form der Modifikation I, **dadurch gekennzeichnet, dass** das Röntgendiffraktogramm der Verbindung Peakmaxima des 2-Theta-Winkels bei 5,9, 6,9, 22,7 aufweist, und einen oder mehrere Stabilisatoren mit einer durchschnittlichen Teilchengröße, ausgedrückt als d50, von 500 nm oder weniger, hergestellt mit den Nanopartikeln von Anspruch 11.

**13.** Pharmazeutische Zusammensetzung nach Anspruch 12, wobei die feste Form aus der Gruppe bestehend aus Granulaten und Tabletten ausgewählt ist.

**14.** Getrocknete Nanopartikel oder pharmazeutische Zusammensetzung nach einem der Ansprüche 11 bis 13, wobei die getrockneten Nanopartikel oder die pharmazeutische Zusammensetzung kein Dispergiermittel enthalten.

## Revendications

**1.** Nanosuspension stable comprenant des nanoparticules de {4,6-diamino-2-[5-fluoro-1-(2-fluorobenzyl)-1H-pyrazolo [3,4-b]pyridin-3-yl]pyrimidin-5-yl}carbamate de méthyle de formule (I)

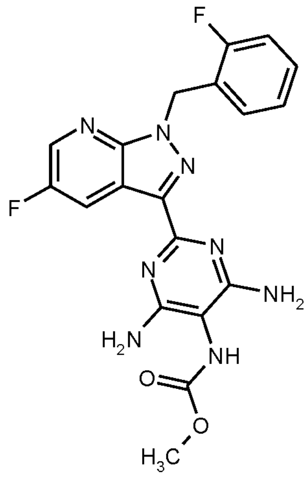

(I)

sous forme cristalline de la modification I, **caractérisée en ce que** le diffractogramme des rayons X du composé présente des maxima de l'angle 2 thêta à 5,9, 6,9, 22,7 et un ou plusieurs stabilisants dans un agent dispersant, dans laquelle le ou les stabilisants sont une polyvinylpyrrolidone (PVP) en combinaison avec le dodécylsulfate de sodium (SDS), ou choisis dans le groupe constitué par un copolymère vinylpyrrolidone-acétate de vinyle, un copolymère séquencé oxyde d'éthylène-oxyde de propylène, le dodécylsulfate de sodium (SDS), l'hydroxypropylméthylcellulose (HPMC), un polysorbate, l'hydroxypropylcellulose (HPC), l'huile de ricin polyoxyl-35, l'hydroxystéarate de polyoxyle 15, le désoxycholate de Na et leurs combinaisons,
la concentration maximale du ou des stabilisants est la limite de solubilité du ou des stabilisants dans la suspension,
l'agent dispersant est choisi dans le groupe constitué par l'eau, les alcools primaires, secondaires et tertiaires, et les alcools polyvalents,
les nanoparticules ont une taille moyenne de particule, exprimée en tant que d50, de 500 nm ou moins, et
la taille moyenne de particule, exprimée en d50, reste à 500 nm ou moins au stockage pendant au moins une semaine à une température d'au moins 40 °C.

**2.** Nanosuspension stable selon la revendication 1, dans laquelle le ou les stabilisants sont choisis dans le groupe constitué par le dodécylsulfate de sodium, une polyvinylpyrrolidone K10 à K50 en combinaison avec le dodécylsulfate de sodium (SDS), l'hydroxypropylméthylcellulose, un polysorbate 20-80, et des combinaisons de ceux-ci.

**3.** Nanosuspension stable selon la revendication 1 ou 2, dans laquelle le rapport du composé de formule (I):un ou plusieurs stabilisants est de 16:1 à 1:2 p/p.

**4.** Nanosuspension stable selon l'une quelconque des revendications 1 à 3, dans laquelle les nanoparticules ont une taille moyenne de particule, exprimée en tant que d50, de 300 nm ou moins.

**5.** Nanosuspension stable selon l'une quelconque des revendications 1 à 4, dans laquelle la taille moyenne de particule, exprimée en tant que d50, reste à 300 nm ou moins au stockage pendant au moins une semaine à une température d'au moins 40 °C.

**6.** Procédé de préparation de la nanosuspension stable selon l'une quelconque des revendications 1 à 5, comprenant les étapes de

a. mise en suspension du composé de formule (I) sous forme cristalline de la modification I dans un agent dispersant choisi dans le groupe constitué par l'eau, les alcools primaires, secondaires et tertiaires et les alcools polyvalents, et un ou plusieurs stabilisants selon la revendication 1 ou 2, dans lequel la concentration maximale du ou des stabilisants est la limite de solubilité du ou des stabilisants dans la suspension ;
b. broyage à billes humide de la suspension générée à l'étape a. avec un apport d'énergie spécifique de 10 000 kJ/kg ou plus et une intensité de contrainte de 0,004 x $10^{-3}$ Nm à 1 $\times$ $10^{-3}$ Nm, les billes de broyage utilisées ayant une taille de 0,05 à 0,8 mm.

**7.** Procédé selon la revendication 6, dans lequel, dans l'étape a., le composé de formule (I) sous forme cristalline de la modification I est micronisé dans une première étape avant de le mettre en suspension dans un agent dispersant et un ou plusieurs stabilisants.

**8.** Procédé selon la revendication 6, dans lequel, dans l'étape a., le composé de formule (I) sous forme cristalline de la modification I est broyé dans une première étape de broyage à billes humide en présence de billes de broyage ayant une taille de 1-2 mm avant de le mettre en suspension dans un agent dispersant et un ou plusieurs stabilisants.

**9.** Procédé selon l'une quelconque des revendications 6 à 8, dans lequel les billes de broyage sont faites d'un matériau choisi dans le groupe choisi parmi les céramiques, le verre, les polymères et l'acier.

**10.** Procédé selon l'une quelconque des revendications 6 à 9, dans lequel le remplissage volumétrique avec des billes de broyage est de 50 à 85 % v/v.

**11.** Nanoparticules séchées contenant du {4,6-diamino-2-[5-fluoro-1-(2-fluorobenzyl)-1H-pyrazolo[3,4-b]pyridin-3-yl] pyrimidin-5-yl}carbamate de méthyle de formule (I) sous forme cristalline de la modification I, **caractérisées en ce que** le diffractogramme des rayons X du composé présente des maxima de l'angle 2 thêta à 5,9, 6,9, 22,7, et un ou plusieurs stabilisant(s) en un rapport du composé de formule (I):un ou plusieurs stabilisant(s) de 16:1 à 1:2 p/p, dans lesquelles les nanoparticules séchées ont une taille moyenne de particule exprimée comme d50 de 500 nm ou moins, le ou les stabilisants sont tels que définis dans la revendication 1, et les nanoparticules séchées sont produites par séchage des nanosuspensions stables préparées par le procédé selon la revendication 6.

**12.** Composition =- pharmaceutique sous forme solide comprenant des nanoparticules séchées de {4,6-diamino-2-[5-fluoro-1-(2-fluorobenzyl)-1H-pyrazolo[3,4-b]pyridin-3-yl]pyrimidin-5-yl}carbamate de méthyle de formule (I) sous forme cristalline de modification I, **caractérisée en ce que** le diffractogramme des rayons X du composé présente des maxima de l'angle 2 thêta à 5,9, 6,9, 22,7, et un ou plusieurs stabilisants ayant une taille moyenne de particules, exprimée comme d50, de 500 nm ou moins, préparée avec les nanoparticules selon la revendication 11.

**13.** Composition pharmaceutique selon la revendication 12, dans laquelle la forme solide est choisie dans le groupe constitué par les granules et les comprimés.

**14.** Nanoparticules séchées ou composition pharmaceutique selon l'une quelconque des revendications 11 à 13, les nanoparticules séchées ou la composition pharmaceutique ne contenant pas un agent dispersant.

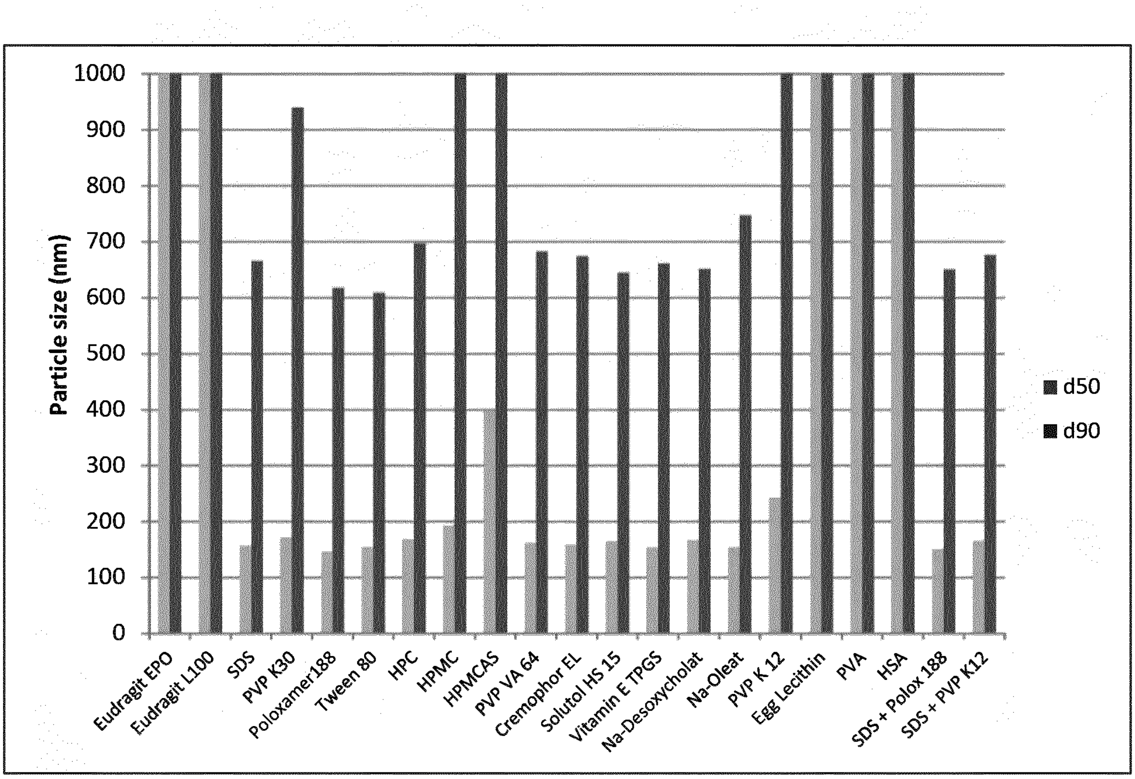
Particle size (nm)
0
100
200
300
400
500
600
700
800
900
1000
Eudragit EPO
Eudragit L100
SDS
PVP K30
Poloxamer188
Tween 80
HPC
HPMC
HPMCAS
PVP VA 64
Cremophor EL
Solutol HS 15
Vitamin E TPGS
Na-Desoxycholat
Na-Oleat
PVP K 12
Egg Lecithin
PVA
HSA
SDS + Polox 188
SDS + PVP K12
d50
d90

Fig. 1a

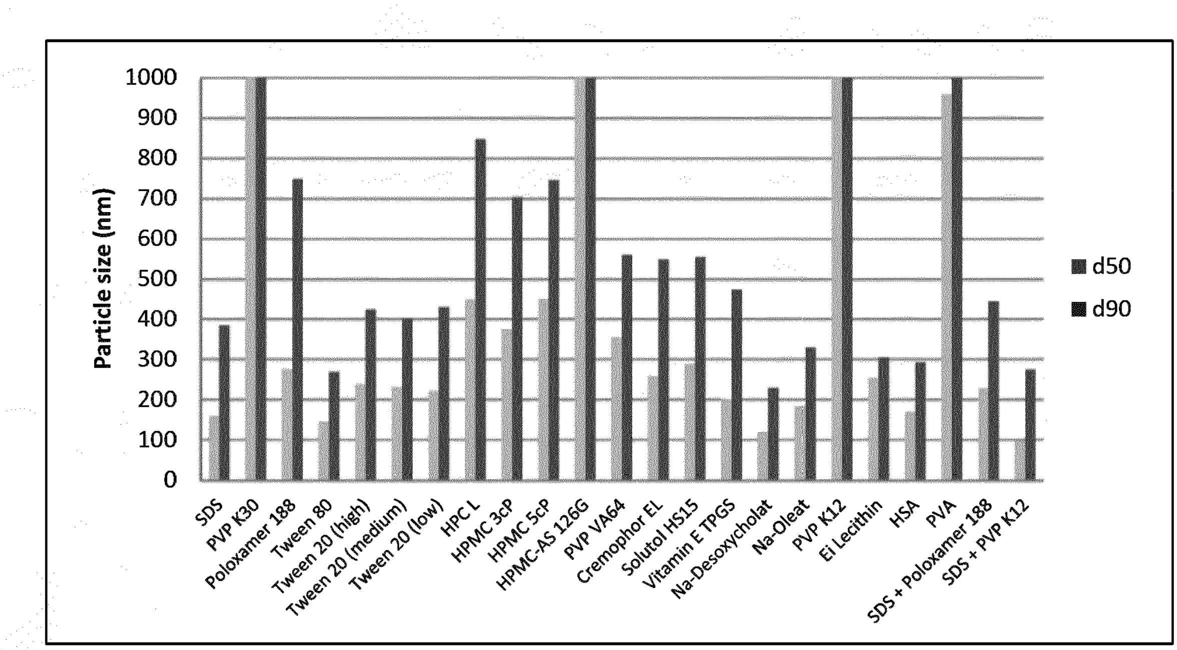
Particle size (nm)
0
100
200
300
400
500
600
700
800
900
1000
SDS
PVP K30
Poloxamer 188
Tween 80
Tween 20 (high)
Tween 20 (medium)
Tween 20 (low)
HPC L
HPMC 3cP
HPMC 5cP
HPMC-AS 126G
PVP VA64
Cremophor EL
Solutol HS15
Vitamin E TPGS
Na-Desoxycholat
Na-Oleat
PVP K12
Ei Lecithin
HSA
PVA
SDS + Poloxamer 188
SDS + PVP K12
d50
d90

Fig. 1b

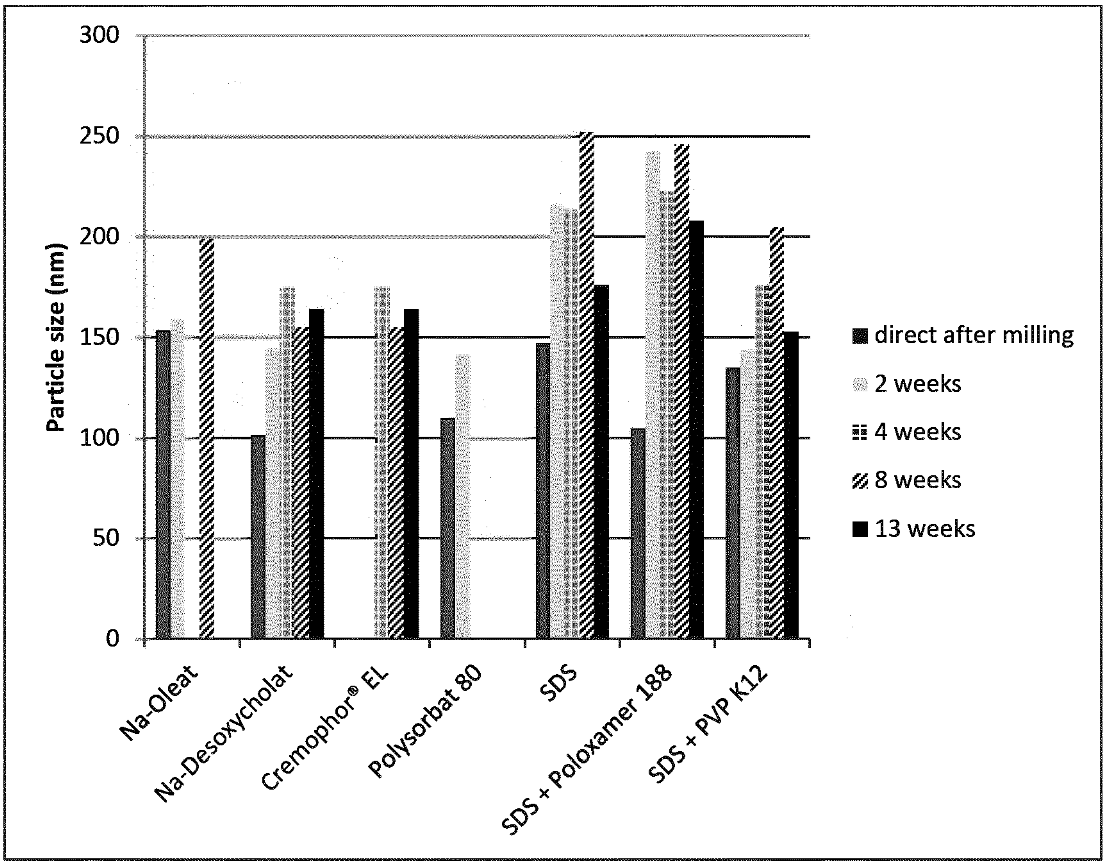

300
250
200
150
100
50
0
Particle size (nm)
Na-Oleat
Na-Desoxycholat
Cremophor® EL
Polysorbat 80
SDS
SDS + Poloxamer 188
SDS + PVP K12
direct after milling
2 weeks
4 weeks
8 weeks
13 weeks


Fig. 2

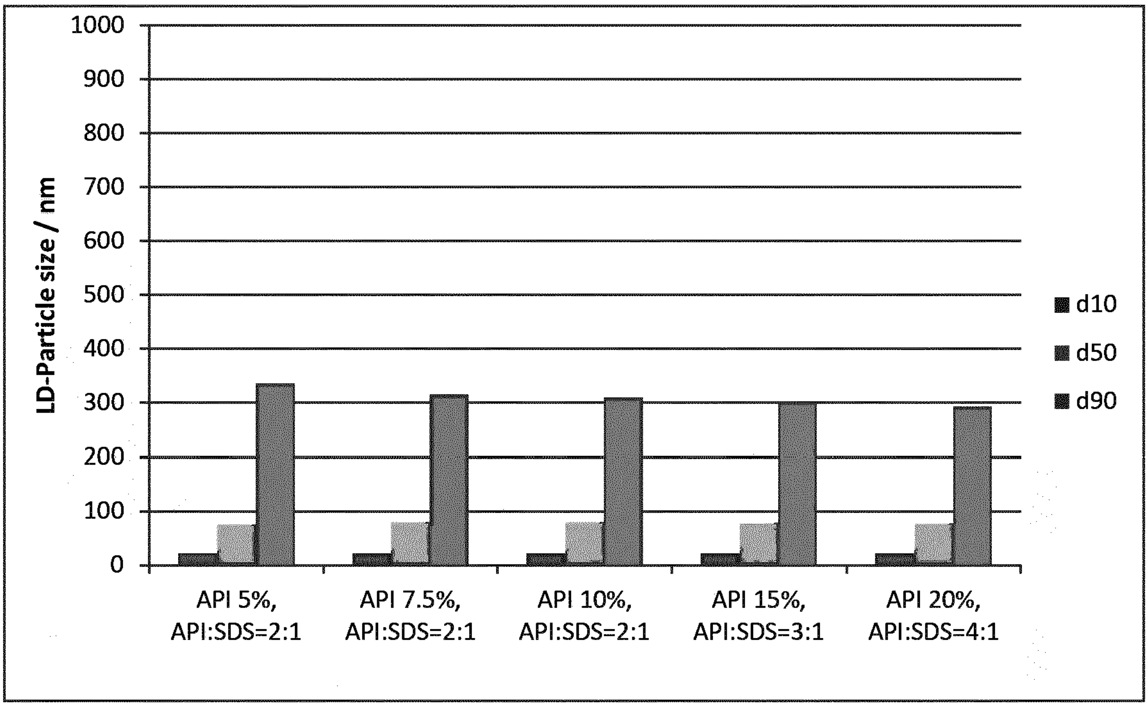

1000
900
800
700
600
500
400
300
200
100
0
LD-Particle size / nm
API 5%, API:SDS=2:1
API 7.5%, API:SDS=2:1
API 10%, API:SDS=2:1
API 15%, API:SDS=3:1
API 20%, API:SDS=4:1
d10
d50
d90


Fig. 3

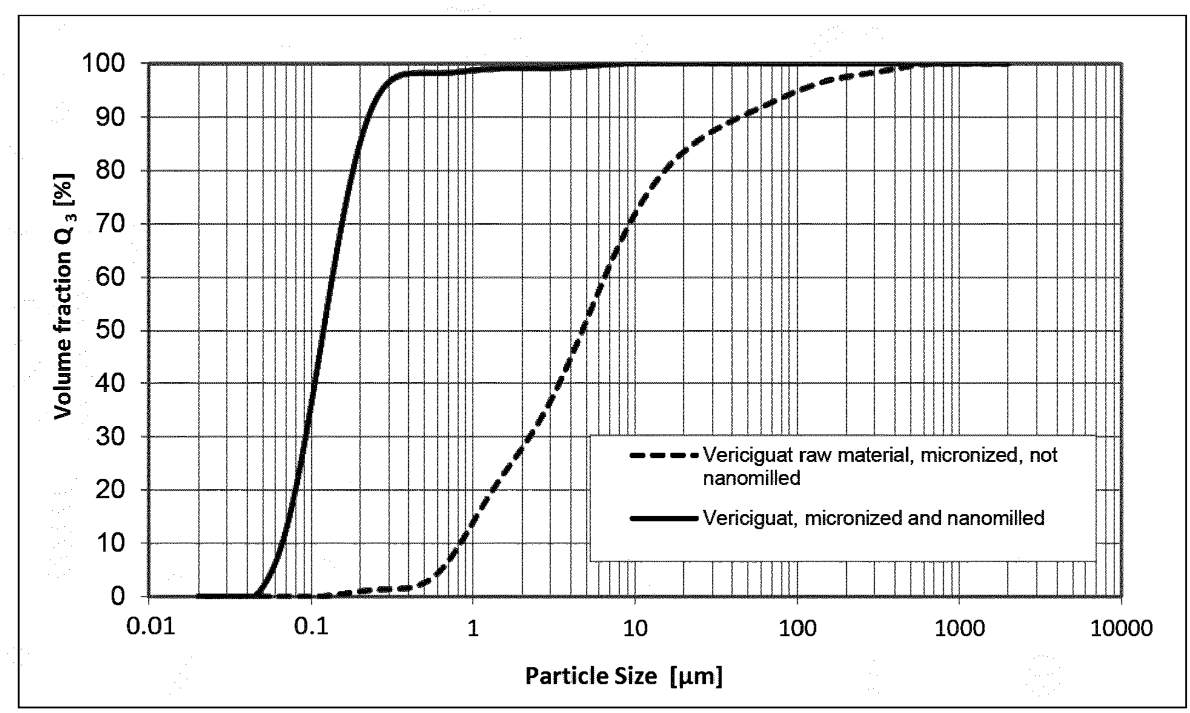
Volume fraction $Q_3$ [%]
Particle Size [µm]
Vericiguat raw material, micronized, not nanomilled
Vericiguat, micronized and nanomilled
0.01
0.1
1
10
100
1000
10000

Fig. 4

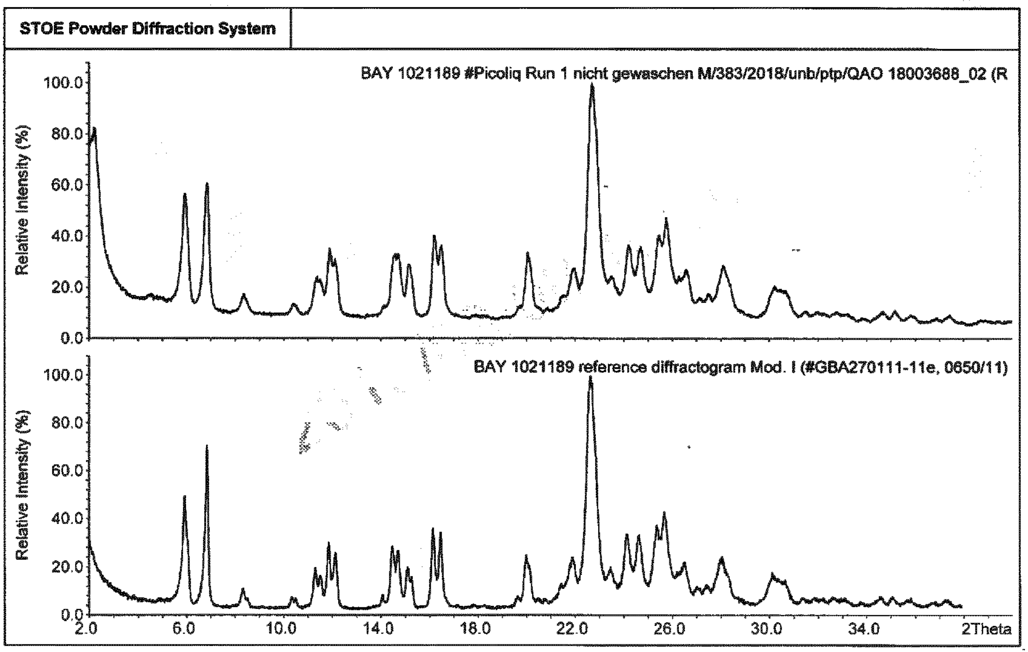
STOE Powder Diffraction System
BAY 1021189 #Picoliq Run 1 nicht gewaschen M/383/2018/unb/ptp/QAO 18003688_02 (R
BAY 1021189 reference diffractogram Mod. I (#GBA270111-11e, 0650/11)
Relative Intensity (%)
2Theta

Fig. 5a

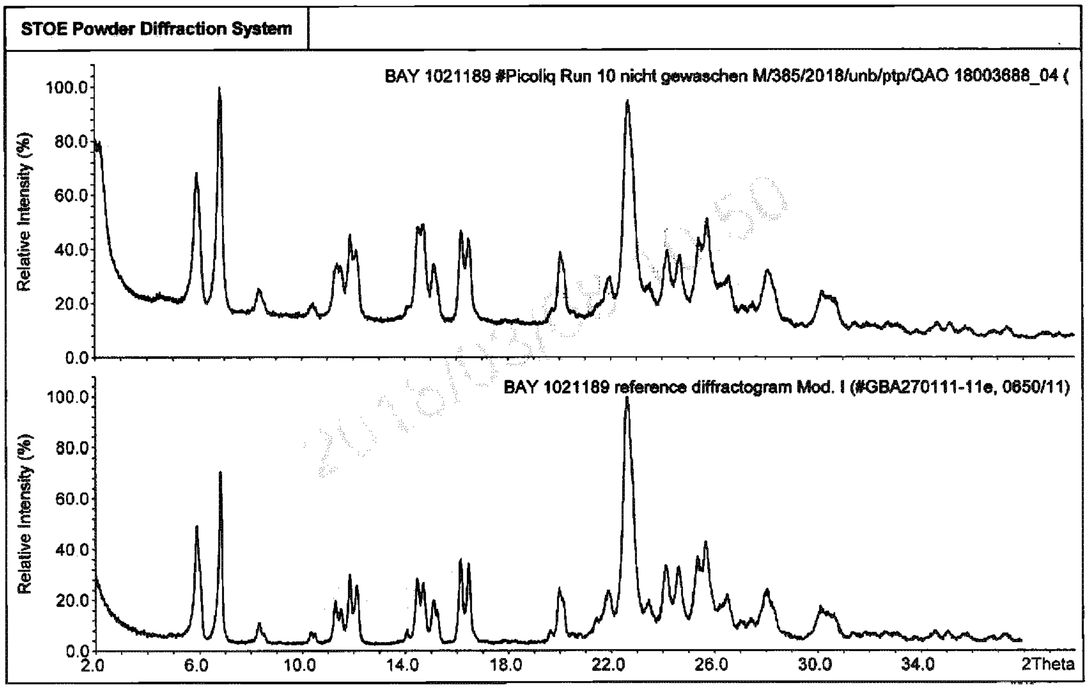
STOE Powder Diffraction System
BAY 1021189 #Picoliq Run 10 nicht gewaschen M/385/2018/unb/ptp/QAO 18003688_04 (
BAY 1021189 reference diffractogram Mod. I (#GBA270111-11e, 0650/11)
Relative Intensity (%)
2Theta

Fig. 5b

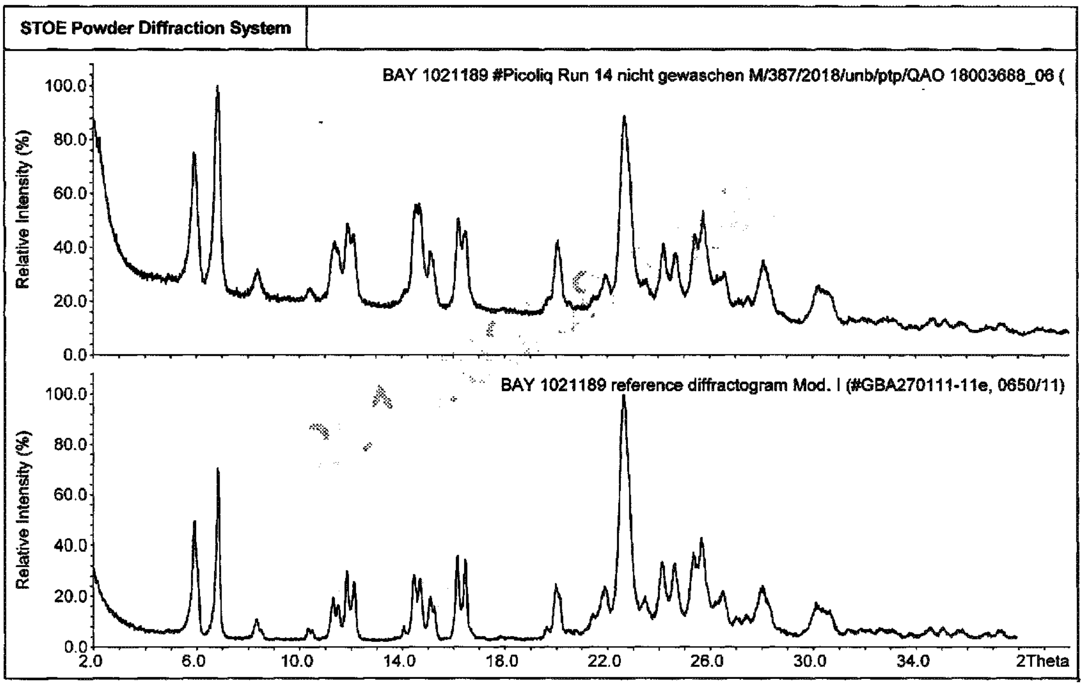
STOE Powder Diffraction System
BAY 1021189 #Picoliq Run 14 nicht gewaschen M/387/2018/unb/ptp/QAO 18003688_06 (
BAY 1021189 reference diffractogram Mod. I (#GBA270111-11e, 0650/11)
Relative Intensity (%)
2Theta

Fig. 5c

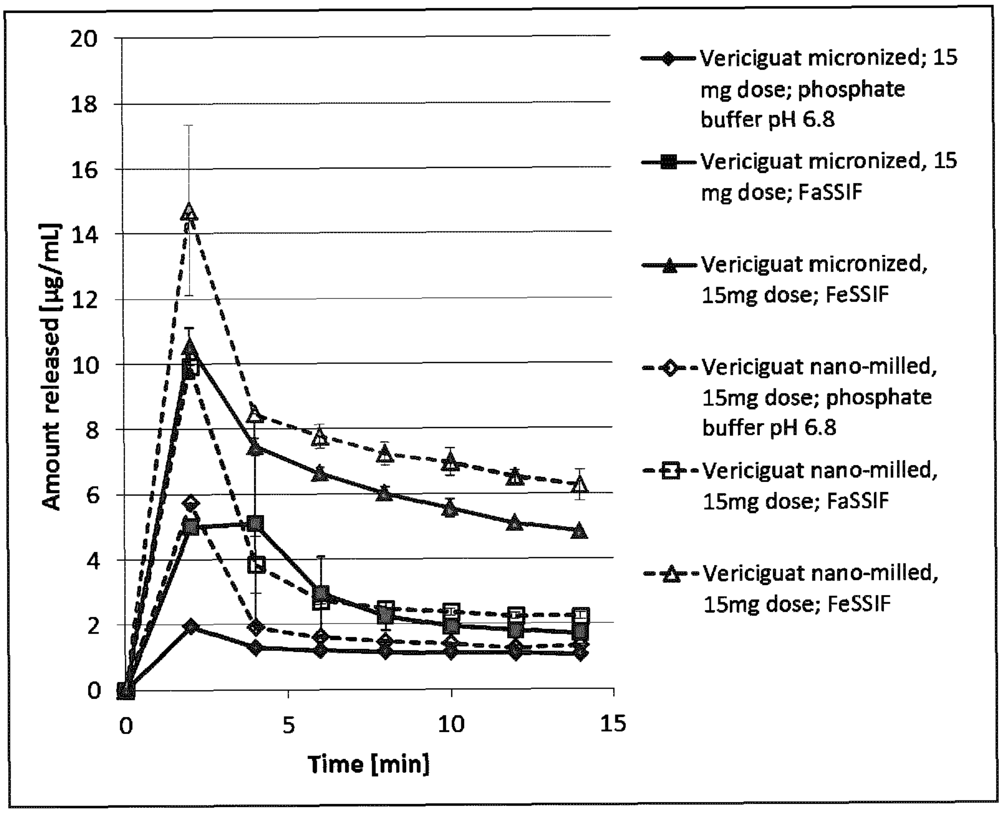
20
18
16
14
12
10
8
6
4
2
0
Amount released [µg/mL]
0
5
10
15
Time [min]
Vericiguat micronized; 15 mg dose; phosphate buffer pH 6.8
Vericiguat micronized, 15 mg dose; FaSSIF
Vericiguat micronized, 15mg dose; FeSSIF
Vericiguat nano-milled, 15mg dose; phosphate buffer pH 6.8
Vericiguat nano-milled, 15mg dose; FaSSIF
Vericiguat nano-milled, 15mg dose; FeSSIF

Fig. 6

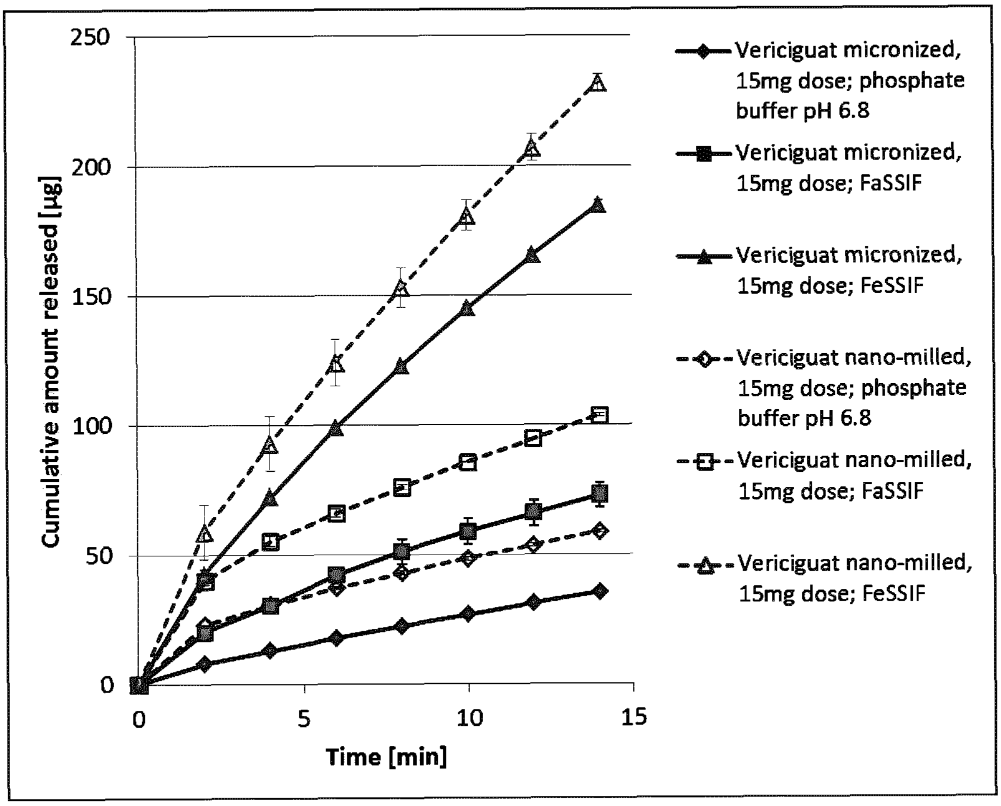
250
200
150
100
50
0
Cumulative amount released [µg]
0
5
10
15
Time [min]
Vericiguat micronized, 15mg dose; phosphate buffer pH 6.8
Vericiguat micronized, 15mg dose; FaSSIF
Vericiguat micronized, 15mg dose; FeSSIF
Vericiguat nano-milled, 15mg dose; phosphate buffer pH 6.8
Vericiguat nano-milled, 15mg dose; FaSSIF
Vericiguat nano-milled, 15mg dose; FeSSIF

Fig. 7

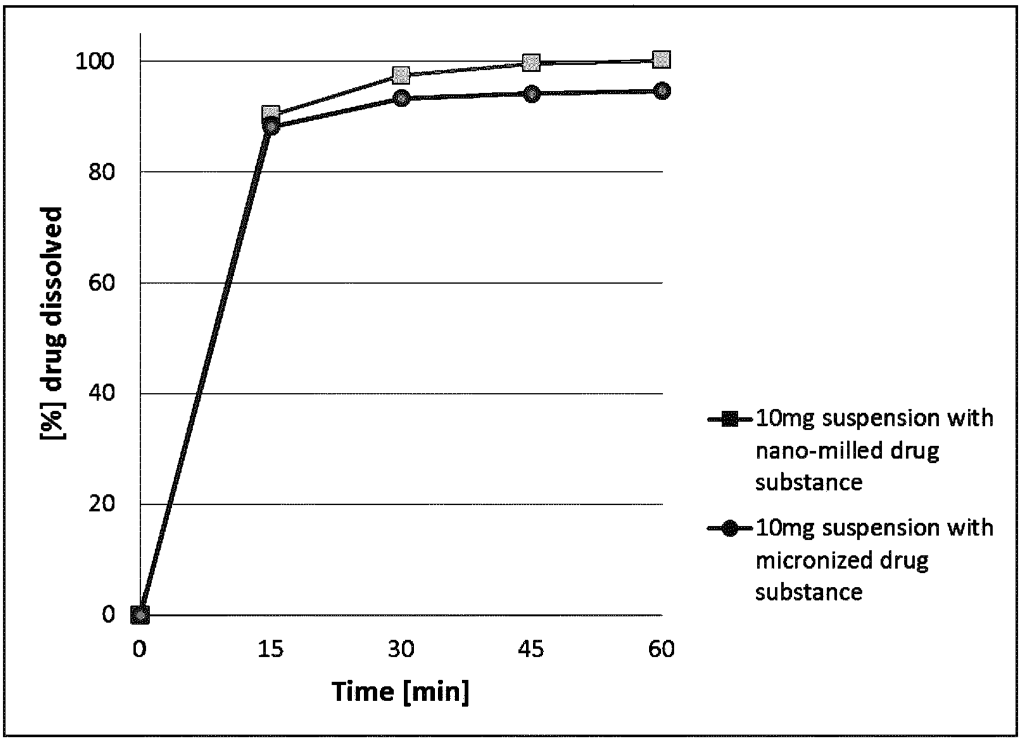
100
80
60
40
20
0
[%] drug dissolved
0
15
30
45
60
Time [min]
10mg suspension with nano-milled drug substance
10mg suspension with micronized drug substance

Fig. 8

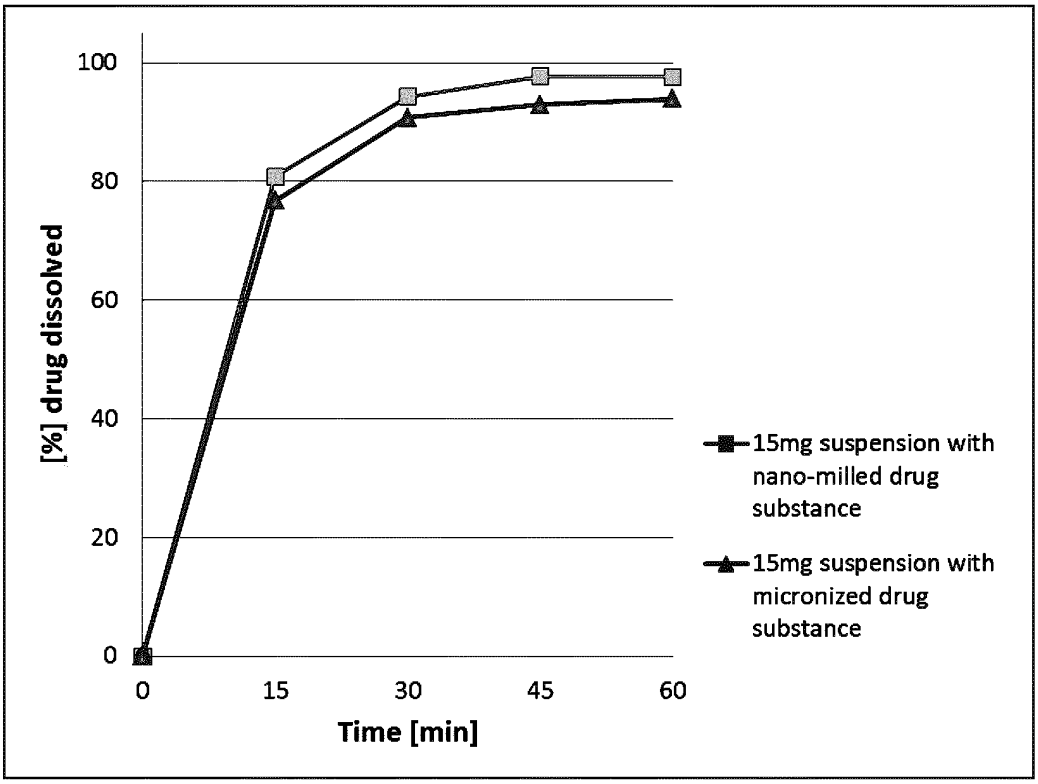
100
80
60
40
20
0
[%] drug dissolved
0
15
30
45
60
Time [min]
15mg suspension with nano-milled drug substance
15mg suspension with micronized drug substance

Fig. 9

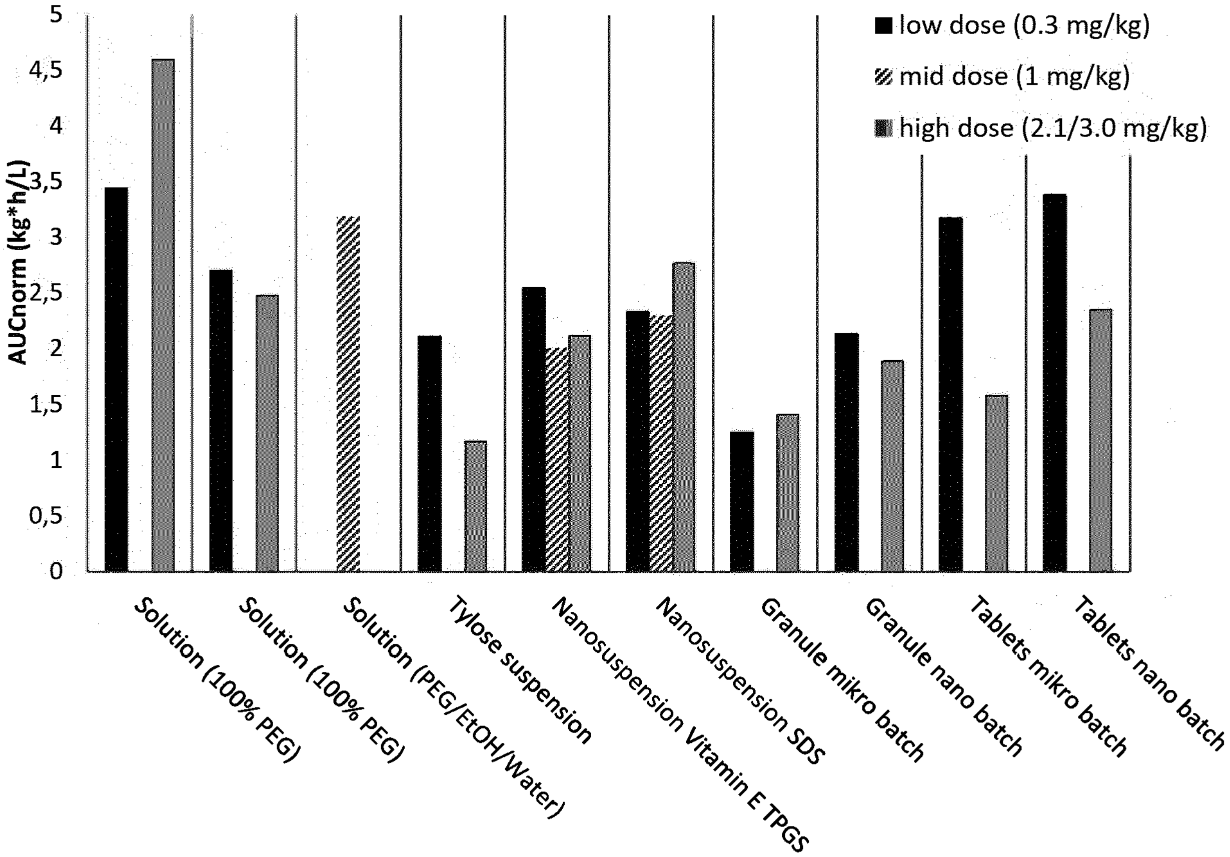
5
4,5
4
3,5
3
2,5
2
1,5
1
0,5
0
AUCnorm (kg*h/L)
low dose (0.3 mg/kg)
mid dose (1 mg/kg)
high dose (2.1/3.0 mg/kg)
Solution (100% PEG)
Solution (100% PEG)
Solution (PEG/EtOH/Water)
Tylose suspension
Nanosuspension Vitamin E TPGS
Nanosuspension SDS
Granule mikro batch
Granule nano batch
Tablets mikro batch
Tablets nano batch

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011147809 A
- WO 2011147809 A1
- WO 2013076168 A1
- WO 2020126983 A1
- WO 2020014504 A1
- CN 108721296 A
- US 5145684 A
- US 8258132 B
- US 6375986 B
- US 7276249 B
- US 7320802 B
- US 6592903 B
- US 9101540 B
- WO 2013076168 A
- WO 2020126983 A


### Non-patent literature cited in the description


- **HUNTER**. Nanomedicine in Health and Disease. 2011
- **BUTLER JM** ; **DRESSMAN JB**. The developability classification system: application of biopharmaceutics concepts to formulation. *J Pharm Sci*, 2010, vol. 99, 4940-4954
- **CHOI YH** ; **HAN H-K**. Nanomedicines: current status and future perspectives in aspect of drug delivery and pharmacokinetics. *Journal of Pharmaceutical Investigation*, 2018, vol. 48, 43-60
- **DESAI PP** ; **DATE AA** ; **PATRAVALE VB**. Overcoming poor oral bioavailability using nanoparticle formulations - opportunities and limitations. *Drug Discovery Today, Technologies*, 2012, vol. 9, e87-e95
- **FOLLMANN M et al.** Discovery of the Soluble Guanylate Cyclase Stimulator Vericiguat (BAY 1021189) for the Treatment of Chronic Heart Failure. *J Med Chem*, 2017, vol. 60, 5146-5161
- **GEORGE M** ; **GOSH I**. Identifying the correlation between drug/stabilizer properties and critical quality attributes (CQAs) of nanosuspension formulation prepared by wet milling technology. *Eur J Phar Sci*, 2013, vol. 48, 142-152
- Nanocrystal Formulations for Improved Delivery of Poorly Soluble Drugs. **HUNTER JR** ; **PREEDY VR**. Nanomedicine in Health and Disease. CRC press, 2011
- **JERMAIN SV** ; **BROUGH, C** ; **WILLIAMS, RO**. Amorphous solid dispersions and nanocrystal technologies for poorly water-soluble drug delivery-An update. *International Journal of Pharmaceutics*, 2018, vol. 535, 379-392
- **KUMAR MP** ; **RAO YM** ; **APTE S**. Formulation of Nanosuspensions of Albendazole for Oral Administration. *Current Nanoscience*, 2008, vol. 4, 53-58
- **KWADE A** ; **BLECHER L** ; **SCHWEDES J**. Motion and stress intensity of grinding beads in a stirred media mill. Part 2: Stress intensity and its effect on comminution. *Powder technology*, 1996, vol. 86, 69-76
- **LI M** ; **AZAD M** ; **DAVÉ R** ; **BILGILI E**. Nanomilling of Drugs for Bioavailability Enhancement: A Holistic Formulation-Process Perspective. *Pharmaceutics*, 2016, vol. 8, 17
- **MERISKO-LIVERSIDGE, EM** ; **LIVERSIDGE, GG**. Drug nanoparticles: formulating poorly water-soluble compounds Toxicol. *Pathol*, 2008, vol. 36, 43-48
- **VAN EERDENBRUGH B** ; **VAN DEN MOOTER G** ; **AUGUSTIJNS P**. Top-down production of drug nanocrystals: Nanosuspension stabilization, miniaturization and transformation into solid products. *Int J Pharm*, 2008, vol. 364, 64-75